(19) Europäisches Patentamt
European Patent Office
Office européen des brevets

(11) EP 0 381 422 B1

(12) EUROPEAN PATENT SPECIFICATION

(45) Date of publication and mention of the grant of the patent: **23.10.1996 Bulletin 1996/43**

(21) Application number: **90300918.1**

(22) Date of filing: **30.01.1990**

(51) Int Cl.[6]: **C07D 403/06**, A61K 31/415, C07D 401/06, C07D 409/06, C07D 413/06, C07D 235/26, C07D 235/30, C07D 401/12, C07D 403/12, C07D 409/12, C07D 417/12

(54) **Tetrahydrobenzimidazole derivatives**

Tetrahydrobenzimidazol-Derivate

Dérivés de tétrahydrobenzimidazole

(84) Designated Contracting States:
**AT BE CH DE DK ES FR GB GR IT LI LU NL SE**

(30) Priority: **02.02.1989 JP 25397/89**
**28.02.1989 JP 48897/89**
**20.10.1989 JP 273444/89**
**28.12.1989 JP 342939/89**

(43) Date of publication of application: **08.08.1990 Bulletin 1990/32**

(73) Proprietor: **YAMANOUCHI PHARMACEUTICAL CO. LTD.**
**Tokyo (JP)**

(72) Inventors:
- **Ohta, Mitsuaki**
**Tsukuba-gun, Ibaraki 300 (JP)**
- **Koide, Tokuo**
**Tsukuba-shi, Ibaraki 305 (JP)**
- **Suzuki, Takeshi**
**Tsukuba-shi, Ibaraki 305 (JP)**
- **Matsuhisa, Akira**
**Tsukuba-shi, Ibaraki 305 (JP)**
- **Miyata, Keiji**
**Shinjuku-ku, Tokyo 161 (JP)**
- **Ohmori, Jun-ya**
**Tsukuba-shi, Ibaraki 305 (JP)**
- **Yanagisawa, Isao**
**Tokyo 177 (JP)**

(74) Representative: **Geering, Keith Edwin et al**
**REDDIE & GROSE**
**16 Theobalds Road**
**London WC1X 8PL (GB)**

(56) References cited:
**EP-A- 0 200 444**
**GB-A- 2 125 398**
**GB-A- 2 166 726**
**GB-A- 2 166 727**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 381 422 B1

## Description

This invention provides tetrahydrobenzimidazole derivative of formula (I) and pharmaceutically acceptable salts thereof :

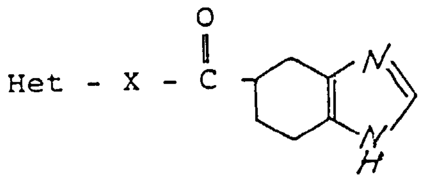

(I)

wherein Het is a heterocyclic group which may be substituted with 1 to 3 substituents selected from lower alkyl, lower alkenyl, lower alkynyl, cycloalkyl-lower alkyl, aralkyl, lower alkoxy, nitro, hydroxyl and lower alkoxycarbonyl groups and halogen atoms; and X is a single bond or -NH- which is bonded to a carbon atom or nitrogen atom of the heterocyclic group.

The compounds of the invention are useful as $5\text{-}HT_3$-receptor antagonists.

Conventionally known antagonists to $5\text{-}HT_3$-receptors include azabicyclo compounds as disclosed in British Patents 2,125,398, 2,166,726, 2,166,727, and 2,126,728 , tetrahydrocarbazole compounds as disclosed in British Patent 2,153,821, and azabicyclo compounds as disclosed in EP 200,444.

The compounds according to the present invention are entirely different in structure from any of the above-described known $5\text{-}HT_3$-receptor antagonists.

In formula (I), the heterocyclic group Het includes residues of monocyclic or condensed heterocyclic rings. Specific examples of the heterocyclic ring are pyrrolidine, piperidine, piperazine, morpholine, pyrrole, furan, thiophene, imidazole, oxazole, thiazole, pyrazole, isoxazole, isothiazole, triazole, thiadiazole, oxadiazole, pyridine, pyridazine, pyrimidine, pyrazine, 4H-cyclopentathiazole, indole, isoindole, 2,3-dihydroindole (indoline), isoindoline, hydroxyindole, indazole, indolizine, benzothiophene, benzofuran, benzothiazole, benzimidazole, benzoxazole, 4,5,6,7-tetrahydrobenzothiophene, 2,3-dihydrobenzimidazol-2-one, quinoline, isoquinoline, 1,2,3,4-tetrahydroquinoline, 1,2,3,4-tetrahydroisoquinoline, 1,4-benzoxazine, phenothiazine, carbazole, β-carboline, etc.

The optional substituent(s), when present, may be at any of the possible positions of the heterocyclic group.

Unless otherwise specified, a "lower" group herein means a straight or branched chain group having from 1 to 6 carbon atoms. Specific examples of the lower alkyl group are methyl, ethyl, propyl, butyl, pentyl, hexyl, isopropyl, isobutyl, t-butyl, isopentyl, t-pentyl, isohexyl groups, etc.

Examples of the "lower alkenyl group" include vinyl, allyl, 1-propenyl, 2-butenyl, isopropenyl groups, etc. Examples of the "lower alkynyl group" include ethynyl, 2-propynyl groups, etc. Examples of the "cycloalkyl-lower alkyl group" include cyclopropylmethyl, cyclopentylmethyl, cyclohexylmethyl, cyclohexylethyl, cycloheptylmethyl groups, etc. Examples of the "aralkyl group" include benzyl, phenethyl groups, etc. Examples of the "lower alkoxy group" include methoxy, ethoxy, propoxy, butoxy, pentyloxy, hexyloxy, isopropoxy, isobutoxy, t-butoxy, isopentyloxy, t-pentyloxy, isohexyloxy, 2-ethylbutoxy groups, etc. Examples of the "lower alkoxycarbonyl group" include methoxycarbonyl, ethoxycarbonyl, propoxycarbonyl, isopropoxycarbonyl, butoxycarbonyl, t-butoxycarbonyl, pentyloxycarbonyl, hexyloxycarbonyl groups, etc.

The halogen atom includes chlorine, bromine, iodine, and fluorine atoms.

Of the compounds (I), preferred are those wherein Het is

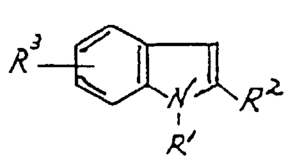

wherein $R^1$ is a hydrogen or halogen atom or a lower alkyl, lower alkenyl, lower alkynyl, cycloalkyl-lower alkyl or aralkyl group; $R^2$ is a hydrogen atom or a lower alkyl or an aralkyl group; and $R^3$ is a hydrogen or-halogen atom or a lower alkoxy, nitro, hydroxyl or lower alkoxycarbonyl group; and X is a single bond. Also preferred are those wherein Het is a nitrogen-containing heterocyclic group and X is a single bond connected to a nitrogen atom of the nitrogen-containing heterocyclic ring.

Also included by the present invention are salts of some of the compounds of formula (I). Examples include salts

with inorganic bases, e.g., sodium and potassium; with organic bases, e.g., ethylamine, propylamine, diethylamine, triethylamine, morpholine, piperidine, N-ethylpiperidine, diethanolamine, and cyclohexylamine; with basic amino acids, e.g., lysine and ornithine;with ammonia;with mineral acids, e.g., hydrochloric, sulfuric, phosphoric and hydrobromic acids; with organic acids, e.g., acetic, oxalic, succinic, citric maleic, malic, fumaric, dibenzoyltartaric , tartaric, and methanesulfonic acids; and with acidic amino acids, e.g., glutamic and aspartic acids.

The present invention further includes tautomers of formula (I) compounds, i.e. compounds of formula:

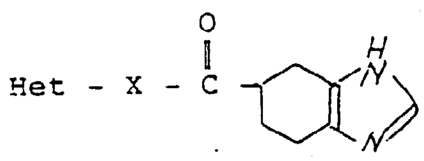

and their pharmaceutically acceptable salts.

Furthermore, the compounds of the present invention have asymmetric carbon atoms, and all the isomers assigned to these asymmetric carbon atoms, such as optically active compounds, racemates, diastereomers, etc., are included by the present invention.

Processes for preparing the compounds according to the present invention are described below.

PROCESS 1 (Amidation):

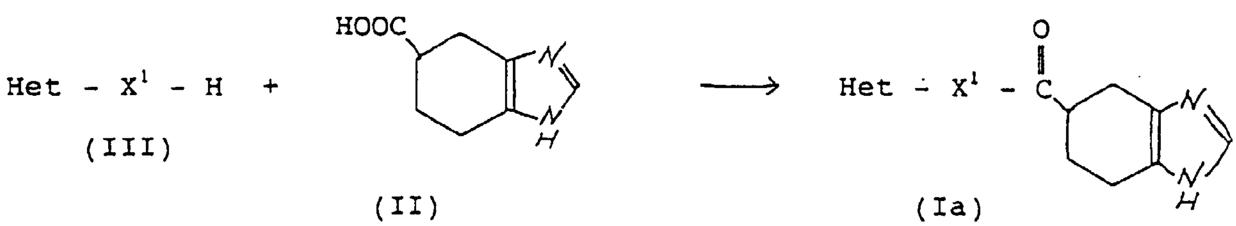

(III) (II) (Ia)

wherein Het is as defined above; and $X^1$ represents a single bond connected to a nitrogen atom of the heterocyclic group, or -NH- connected to a carbon atom of the heterocyclic group.

Compound (Ia) can be obtained by reacting amine, amide, or urea (III) with 4,5,6,7-tetrahydrobenzimidazole-5-carboxylic acid (II) or reactive derivative thereof.

The reaction can be carried out by any of various known processes for amide linkage formation. Suitable solvents are not particularly limited and include dioxane, diethyl ether, tetrahydrofuran, chloroform, ethyl acetate, and dimethylformamide.

The compound (II) may be the free acid or a reactive derivative thereof, e.g., an acid halide, acid anhydride, acid azide, and various active esters generally used in peptide syntheses. In the former case, the amide linkage formation can be effected using any commonly employed condensing agent, for example, N,N-dicyclohexylcarbodiimide.

In some cases ,depending on the kind of reactive derivative (II), the reaction is preferably carried out in the presence of a base, such as inorganic base, e.g., sodium or potassium hydrogencarbonate or carbonate; or organic base. e.g., triethylamine, diisopropylethylamine, dimethylaniline, or pyridine.

Compound (III) is usually used in free form or, if-desired, after conversion to an alkali metal salt.

Compound (III) is desirably used in an equimolar or excessive amount with respect to compound (II) or its reactive derivative.

The reaction may be carried out at room temperature, under cooling, or under heating, depending on the kind of amide linkage reaction, but is usually at room temperature or under cooling.

PROCESS 2:

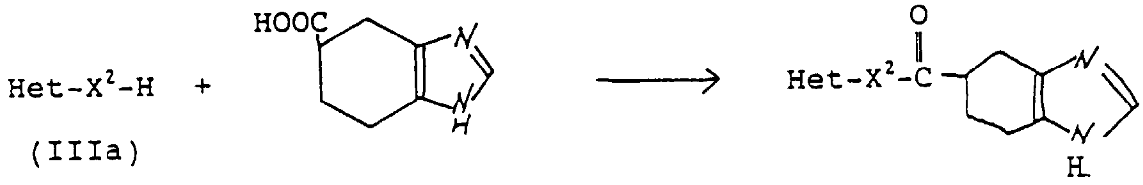

(IIIa) (II) (Ib)

wherein Het is as defined above; and $X^2$ is a single bond connected to a carbon atom of the heterocyclic ring.

Compound (Ib) can be obtained by reacting heterocyclic compound (IIIa) with carboxylic acid (II) or reactive derivative thereof.

The reaction can be carried out by any of various known processes for synthesizing carbonyl compounds using a carboxylic acid or a derivative thereof.

Where a carboxylic acid (II) is employed, the reaction with compound (IIIa) is a dehydrating condensation reaction, e.g. using polyphosphoric acid as a condensing agent. The reaction is carried out with or without a solvent. Solvents which can be used are not limited as long as they are inert to the reaction, but, usually, solvents of appropriate boiling point are selected taking the reaction temperature into consideration. Examples of suitable solvents are decalin, tetralin, diglyme, etc. The reaction oan be at room temperature or preferably under heating.

Where an acid halide of carboxylic acid (II) is employed, the reaction is a Friedel-Crafts reaction which can be carried out by known processes or various modifications thereof using a Lewis acid, e.g., aluminum chloride, ferric chloride, stannic chloride, boron trifluoride ethyl etherate, and titanium tetrachloride. Solvents inert to the reaction may be employed preferably being selected depending on the kind of Lewis acid used. Examples of usable solvents are acetonitrile and carbon disulfide. The reaction oan be at room temperature or, usually, under heating.

Where an acid amide of carboxylic acid (II) is employed, the reaction is a Vilsmeyer reaction, a known reaction frequently employed for synthesis of heterocyclic carbonyl compounds. Reagents for converting the acid amide to a Vilsmeyer complex include general halogenating agents, e.g., phosphorus pentachloride and phosphorus oxychloride. This reaction may be effected with or without a solvent; various kinds of solvents can be employed as long as they are inert to the reaction - an example is 1,2-dichloroethane. The reaction can be at room temperature or under heating, preferably under heating.

PROCESS 3 (N-Alkylation):

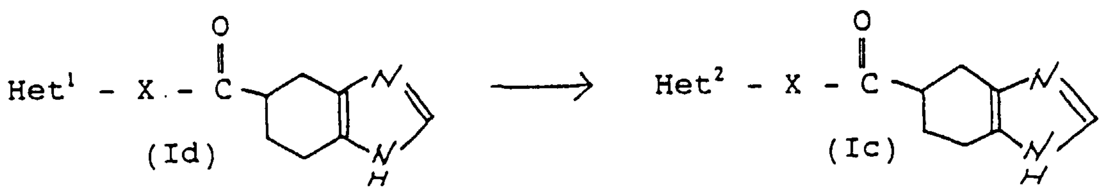

(Id) (Ic)

wherein X is as defined above; $Het^1$ is a heterocyclic group having -NH- in its ring; and $Het^2$ represents a heterocyclic group in which the -NH- moiety in $Het^1$ is converted to

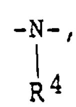

wherein $R^4$ represents a lower alkyl, lower alkenyl, lower alkynyl, cycloalkyl-lower alkyl or aralkyl group.

This reaction is an N-alkylation reaction. The terminology "alkylation" as used herein means introduction of a lower alkyl, lower alkenyl, lower alkynyl, cycloalkyl-lower alkyl or aralkyl group. Various known alkylation techniques are applicable. For example, when the alkylation is carried out by direct N-alkylation using an alkylating agent, the reaction is conducted under cooling, at room temperature, or under heating, preferably under cooling or at room temperature.

Any solvent inert to the reaction, e.g., dioxane and dimethylformamide, may be employed. The reaction is effected in the presence of a base or using an alkali metal salt of compound (Id) at the amino group thereof. Examples of suitable alkylating agents include alkyl halides and sulfates. Examples of suitable bases include inorganic bases, e.g., sodium hydride, and sodium and potassium hydrogen-carbonates and carbonates; and organic bases, e.g., triethylamine, diisopropylamine, dimethylaniline, and pyridine.

The compounds of the present invention can be isolated and purified in free form or in the form of a salt through usual chemical means, such as extraction, crystallization, recrystallization, and various chromatographic techniques.

Stereochemically pure isomers can be obtained by using an appropriate starting compound or by general resolution techniques (for example, by obtaining a diastereomer salt with an ordinary optically active acid, e.g., dibenzoyl tartaric acid, followed by optical resolution).

The compounds according to the present invention specifically inhibit transient bradycardia induced by serotonin in anesthetized rats as demonstrated by Test Example 1 and thus have antagonism against 5-$HT_3$-receptor. Therefore, the compounds of the present invention and the salts thereof are considered to suppress vomiting induced by anticancer agents, e.g., Cisplatin, or radiation and to be useful in the prevention and treatment of migraine, cluster headache, trigeminal neuralgia, anxiety, psychotic disorder, gastro intestinal disorders, peptic ulcer, irritable bowel syndrome, etc.

A pharmaceutical composition containing at least one of the compounds of the present invention as an active ingredient can be prepared in various dose forms, such as tablets, powders, granules, capsules, pills, liquids, injections, suppositories, ointments, pastes, and the like using carriers, excipients and other additives conventionally used in pharmaceuticals. The preparation may be administered orally, inclusive of sublingual administration, or parenterally.

Carriers or excipients for pharmaceutical compositions include solid or liquid non-toxic pharmaceutically acceptable materials, e.g., lactose, magnesium stearate, starch, talc, gelatin, agar, pectin, gum arabic, olive oil, sesame oil, cocoa butter, ethylene glycol, and the like.

The clinical dose of compound of the present invention is determined taking into account condition, body weight, age, sex, etc. of the patient. For adults it is suitably from 0.1 to 10 mg/day for intravenous administration and from 0.5 to 50 mg/day for oral administration, in a single dose or a plurality of divided doses.

The pharmacological effects of compounds of the present invention were confirmed by Test Examples.

TEST EXAMPLE 1

Antagonism Against 5-$HT_3$-Receptor

Nine-week-old Wistar male rats were anesthetized by intravenous injection of 1 g/kg of urethane, and blood pressure and heart rate were measured under artificial respiration. Transient reduction in heart rate and blood pressure induced by intravenous administration of serotonin or 2-methylserotonin which is a selective agonist of 5-$HT_3$ was taken as an index of the reaction via 5-$HT_3$ receptor [Bezold-Jarish reflex; Paintal, A.S., Pysiolo. Rev., Vol. 53, p. 159 (1973)].

When compound of the present invention was administered intravenously (0.03 to 3 μg/kg) or orally (1 to 30 μg/kg) 10 minutes or 60 minutes before the serotonin (or 2-methylserotonin), respectively, the reduction in heart rate and blood pressure induced by serotonin or 2-methylserotonine was dose-dependently inhibited.

Inhibitory activity of compounds of the present invention on serotonin-induced Bezold-Jarish (BJ) reflex in rats is shown in Table 1.

TABLE 1

| Example No. of Test Compound | BJ Reflex Inhibitory Activity ($ED_{50}$; μg/kg, i.v.) |
|---|---|
| 2 | 0.29 |
| 4 | 0.044 |
| 9 | 0.80 |
| 36 | 0.063 |

TEST EXAMPLE 2

Inhibition of Anticancer Agent-Induced Vomiting

When male ferrets weighing from 1 to 1.5 kg subcutaneously or orally received 0.01 to 0.3 mg/kg of compound of the present invention, vomiting induced by intraperitoneal administration of 10 mg/kg of Cisplatin was inhibited.

TEST EXAMPLE 3

Inhibition of Stress Defecation

Nine-week-old male Wistar rats were encased in a cage for restricted stress, and the number of feces was measured. Intravenous administration of compound of the present invention (1 to 100 μg/kg) dose-dependently inhibited acceleration of defecation induced by restricted stress.

TEST EXAMPLE 4

Toxicity

Acute toxicity of compounds of the present invention in male mice was from 100 to 150 mg/kg i.v. as determined by an up-and-down method, indicating that the compounds are of low toxicity.

The present invention is illustrated in greater detail by the following Reference Examples, Examples, and Formulation Examples, but it should be understood that the invention is not limited thereto.

REFERENCE EXAMPLE 1

(a)

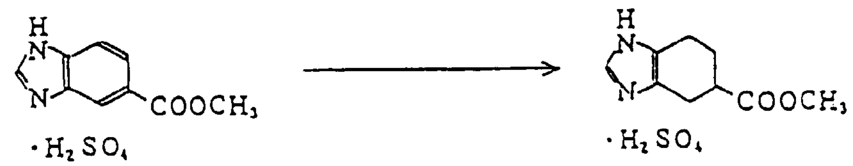

In 600 mℓ of acetic acid was dissolved 40.0 g of methyl 5-benzimidazole carboxylate sulfate in a 1 ℓ-volume autoclave, and 11 g of 10% palladium-on-carbon was added to the solution as a catalyst to conduct hydrogenation at 80°C at a pressure of 60 atm for 5 hours. The catalyst was separated by filtration, and the mother liquor was concentrated under reduced pressure to obtain 41.0 g of methyl 4,5,6,7-tetrahydrobenzimidazole-5-carboxylate sulfate as an oil.

(b)

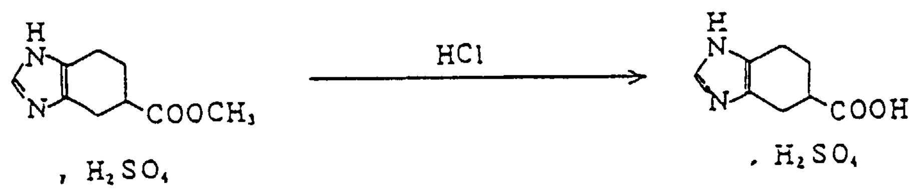


In a mixture of 350 mℓ of water and 340 mℓ of concentrated hydrochloric acid was dissolved 41.0 g of the oily ester sulfate obtained at (a) above, and the mixture was stirred at 100°C for 3 hours. After concentration, the resulting crystals were washed with acetone to obtain 29.6 g (76.8% based on the benzimidazole ester) of 4,5,6,7-tetrahydrobenzimidazole-5-carboxylic acid sulfate.

Physicochemical Properties:

| | |
|---|---|
| Melting Point: | 145-148°C |
| NMR ($d_6$-DMSO): | δ 1.60-3.00 (7H, m), 8.84 (1H, s) |
| Mass Spectrum (EI): | m/z 166 ($M^+$, as a free compound) |
| (CI): | m/z 167 ($M^+$+1, as a free compound) |

REFERENCE EXAMPLE 2

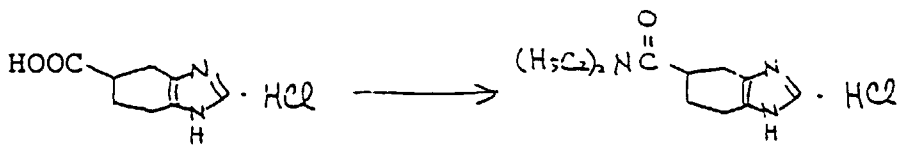

To 0.30 g of 4,5,6,7-tetrahydrobenzimidazole-5-carboxylic acid hydrochloride containing sodium chloride was added 5 mℓ of thionyl chloride, followed by stirring at 90°C for 2 hours. The excess of thionyl chloride was removed by distillation under reduced pressure. To the residue was added 10 mℓ of dichloromethane, and 2 mℓ of diethylamine was added thereto at 5°C, followed by stirring at room temperature for 16 hours. To the mixture was added 40 mℓ of dichloromethane, and the mixture was washed with a saturated aqueous solution of sodium hydrogencarbonate and dried over anhydrous magnesium sulfate. The solvent was removed by distillation under reduced pressure to obtain 0.22 g of N,N-diethyl-4,5,6,7-tetrahydrobenzimidazole-5-carboxamide.

Physicochemical Properties:

| | |
|---|---|
| NMR (TMS, $CDC\ell_3$) δ | 1.15 (t, 6H), 2.0-3.5 (m, 7H), 3.10 (q, 4H), 8.15 (s, 1H), 9.50 (s, 1H) |
| Mass Spectrum (FAB, Pos) | m/z 222 ($M^+$+1) |

To the above obtained compound was added 1 mℓ of a 4N solution of hydrogen chloride in ethyl acetate, and the solvent was removed by distillation under reduced pressure to obtain 0.27 g of N,N-diethyl-4,5,6,7-tetrahydrobenzimidazole-5-carboxamide hydrochloride.

EXAMPLE 1

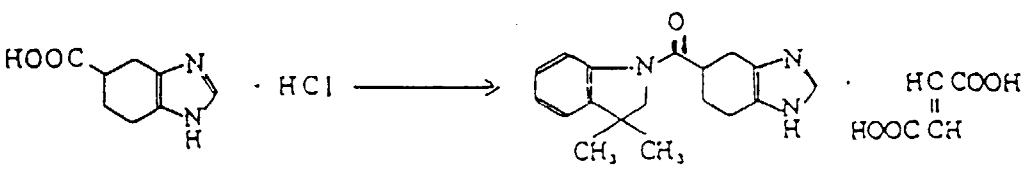

In 0.7 mℓ of thionyl chloride, 0.13 g of 4,5,6,7-tetrahydrobenzimidazole-5-carboxylic acid hydrochloride (containing sodium chloride) was refluxed for 30 minutes, and volatile components were removed by distillation under reduced pressure. The residue was added to a solution of 0.15 g of 3,3-dimethylindoline and 0.15 mℓ of triethylamine in 2 mℓ of dichloromethane under ice-cooling. After stirring the mixture at room temperature overnight, 5 mℓ of a sodium carbonate aqueous solution was added thereto, and the mixture was extracted with chloroform. The organic layer was dried, and the solvent was removed by distillation under reduced pressure. The residue was subjected to silica gel column chromatography using chloroform/methanol as an eluent to obtain 0.11 g of 5-[(2,3-dihydro-3,3-dimethylindol-1-yl)carbonyl] 4,5,6,7-tetrahydrobenzimidazole as an oily substance. The oily substance was then treated with a solution of fumaric acid in methanol/acetonitrile to obtain 0.09 g of 5-[(2,3-dihydro-3,3-dimethylindol-1-yl)carbonyl]-4,5,6,7-tetrahydrobenzimidazole fumarate.

Physicochemical Properties:

Melting Point: 119-123°C

| Elemental Analysis for $C_{18}H_{21}N_3O.C_4H_4O_4.H_2O.0.3CH_3CN$: | | | |
|---|---|---|---|
| Calcd. (%): | C 61.44; | H 6.36; | N 10.46 |
| Found (%): | C 61.60; | H 6.03; | N 10.46 |

Mass Spectrum (EI): m/z 295 ($M^+$, as a free compound)

In the same manner as in Example 1, the following compounds were synthesized.

EXAMPLE 2

5-[(2,3-Dihydroindol-1-yl)carbonyl]-4,5,6,7-tetrahydrobenzimidazole fumarate

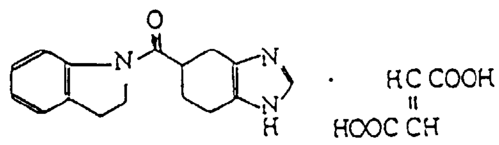

Physicochemical Properties:

Melting Point: 206-208°C (methanol/acetonitrile)

| Elemental Analysis for $C_{16}H_{17}N_3O.C_4H_4O_4.0.3H_2O$: | | | |
|---|---|---|---|
| Calcd. (%): | C 61.78; | H 5.60; | N 10.81 |
| Found (%): | C 61.92; | H 5.53; | N 10.68 |

Mass Spectrum (EI): m/z 267 ($M^+$, as a free compound)

EXAMPLE 3

5-[(2-Methyl-2,3-dihydroindol-1-yl)-carbonyl]-4,5,6,7-tetrahydrobenzimidazole

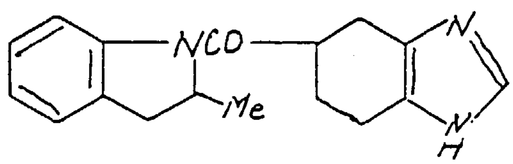

Physicochemical Properties:

Melting Point: 230-234°C (dec.) (recrystallized from ethyl acetate/hexane)

| Elemental Analysis for $C_{17}H_{19}N_3O$: | | | |
|---|---|---|---|
| Calcd. (%): | C 72.57; | H 6.81; | N 14.93 |
| Found (%): | C 72.76; | H 6.78; | N 14.62 |

Mass Spectrum: m/z 281 ($M^+$)

EXAMPLE 4

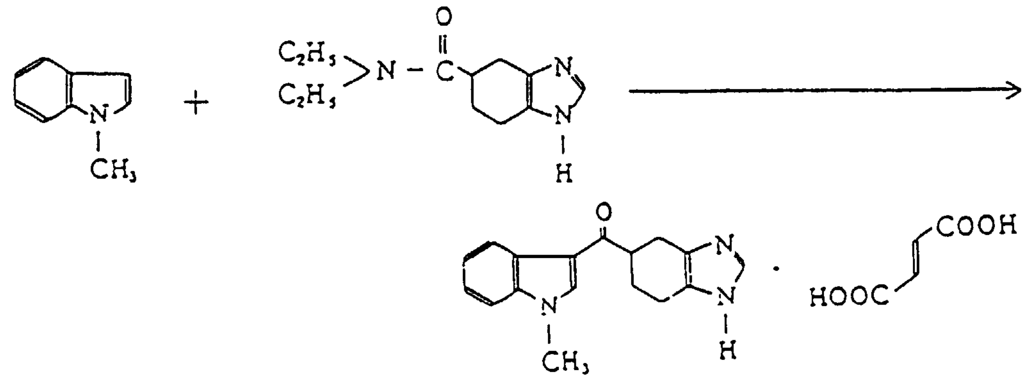

A mixture of 0.27 g (1.05 mmol) of N,N-diethyl 4,5,6,7-tetrahydrobenzimidazole-5-carboxamide hydrochloride, 0.16 mℓ (1.25 mmol) of 1-methylindole, and 0.15 mℓ (1.65 mmol) of phosphorus oxychloride was heated at 80°C for 2 hours while stirring. 30 ml of water were added thereto, and the mixture was rendered basic with 1N sodium hydroxide aqueous solution, followed by extraction with ethyl acetate. The ethyl acetate layer was dried over anhydrous magnesium sulfate and filtered. The filtrate was distilled under reduced pressure, and the residue was purified by silica gel column chromatography (eluent: dichloromethane/methanol/aqueous ammonia = 10:1:0.1 by volume) and preparative thin layer chromatography (developing solvent : dichloromethane/methanol/aqueous ammonia = 10:1: 0.1 by volume) to obtain 20 mg of a foam substance. To the product was added 10 mg of fumaric acid to convert it to a fumarate. Recrystallization from ethyl acetate/methanol (10:1 by volume; gave 10 mg of 5-[(1-methylindol-3-yl)carbonyl]-4,5,6,7-tetrahydrobenzimidazole fumarate.

Physicochemical Properties:

| | |
|---|---|
| Melting Point: | 97-102°C |
| Mass Spectrum (EI): | m/z 279 ($M^+$, as a free compound) |
| NMR ($CDC\ell_3$) δ (as a free compound): | 1.90-3.00 (7H, m, $CH_2$, CH), 3.80 (3H, s, N-Me), 7.20 (2H, m, ArH), 7.50-8.00 (4H, m, ArH), 8.30 (1H, m, NH) |

Example 5

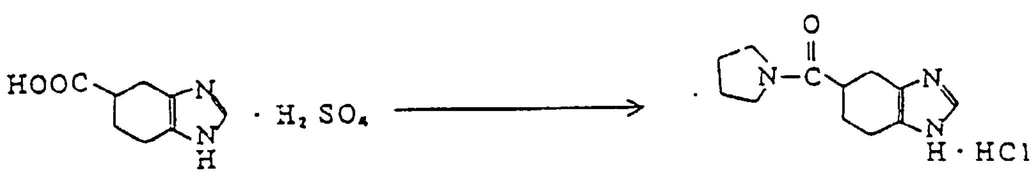

To 53 mℓ of acetonitrile were added 5.3 g of 4,5,6,7-tetrahydrobenzimidazole-5-carboxylic acid sulfate and 2.9 mℓ of thionyl chloride, and the mixture was stirred at 53 to 55°C for 1.5 hours. The mixture was distilled under reduced pressure to remove 10 to 15 mℓ of the solvent. After 15 mℓ of acetonitrile was added thereto, the mixture was further distilled under reduced pressure to remove 10 to 15 mℓ of the solvent. The residual solution was added dropwise to a solution of 14.2 g of pyrrolidine in 50 mℓ of acetonitrile at 2°C or lower. After the addition, the temperature was returned to room temperature, and the mixture was stirred for 1 hour, followed by concentration under reduced pressure. To the residue was added 30 mℓ of saturated sodium chloride aqueous solution, and the mixture was extracted with chloroform (50 mℓ x 3). The chloroform layer was dried over anhydrous magnesium sulfate, concentrated under reduced pressure, treated with hydrochloric acid in ethanol, and recrystallized from ethanol/ethyl acetate to obtain 4.25 g (82.9%) of N-[(4,5,6,7-tetrahydrobenzimidazol-5-yl)carbonyl]pyrrolidine hydrochloride.

Physicochemical Properties:

Melting Point: 234-236°C

| Elemental Analysis for $C_{12}H_{18}N_3OC\ell.0.2H_2O$: | | | | |
|---|---|---|---|---|
| Calcd. (%): | C 55.57; | H 7.15; | N 16.20; | Cℓ 13.67 |
| Found (%): | C 55.64; | H 6.99; | N 16.18; | Cℓ 13.79 |

Mass Spectrum (EI): m/z 291 ($M^+$, as a free compound)

In the same manner as in Example 5, the following compounds was synthesized.

EXAMPLE 6

4-(4,5,6,7-Tetrahydrobenzimidazol-5-ylcarbonyl)-2,3-dihydro-1,4-benzoxazine fumarate

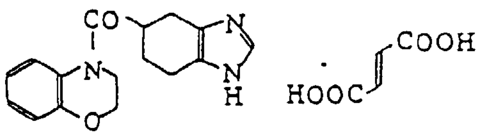

Physicochemical Properties:

Melting Point: 176-178°C (methanol/acetonitrile)

Mass Spectrum (EI): m/z 283 ($M^+$, as a free compound)

| Elemental Analysis for $C_{16}H_{17}N_3O_2.C_4H_4O_4$: | | | |
|---|---|---|---|
| Calcd. (%): | C 60.14; | H 5.30; | N 10.52 |
| Found (%): | C 59.95; | H 5.28; | N 10.55 |

EXAMPLE 7

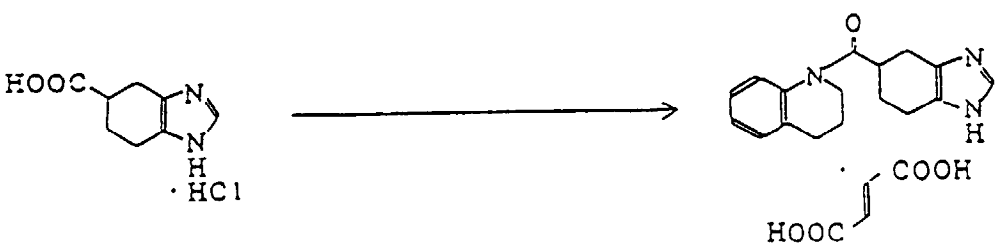

5 mℓ of thionyl chloride was added 0.58 g (0.98 mmol) of 4,5,6,7-tetrahydrobenzimidazole-5-carboxylic acid hydrochloride having a purity of 34.5% (containing sodium chloride), and the mixture was stirred at 90°C for 4 hours. After cooling, the solution was distilled under reduced pressure to remove thionyl chloride. To the residue was added 10 mℓ of dichloromethane, and 0.20 mℓ (1.59 mmol) of 1,2,3,4-tetrahydroquinoline and 0.35 mℓ (2.53 mmol) of triethylamine were added thereto, followed by stirring at room temperature for 48 hours. To the reaction mixture was added 40 mℓ of dichloromethane, and the mixture was washed with 1N sodium hydroxide aqueous solution and dried over anhydrous magnesium sulfate. The solvent was removed by distillation under reduced pressure, and the residue was subjected to silica gel column chromatography using dichloromethane/methanol/aqueous ammonia (10:1:0.1 by volume) as an eluent to obtain 100 mg of a foam substance, which was then treated with 40 mg of fumaric acid in ethanol to be converted to a fumarate. Recrystallization from ethyl acetate/methanol (10:1 by volume) gave 90 mg (33.3%) of 1-[(4,5,6,7-tetrahydrobenzimidazol-5-yl)carbonyl]-1,2,3,4-tetrahydroquinoline fumarate.

Physicochemical Properties:

Melting Point: 98-100°C

| Elemental Analysis for $C_{17}H_{19}N_3O.C_4H_4O_4.2H_2O$: | | | |
|---|---|---|---|
| Calcd. (%): | C 58.19; | H 6.27; | N 9.69 |
| Found (%): | C 58.43; | H 5.73; | N 9.53 |

NMR (DMSO-$d_6$) δ ppm: 1.90 (4H, q, 7Hz, quinoline $CH_2$x2), 2.00-3.00 (7H, m, benzimidazole $CH_2$x3, CH), 3.70 (2H, t, J=7Hz, $CH_2N$), 6.60 (2H, s, fumaric acid CHx2), 7.16 (5H, m, ArH, NH), 7.55 (1H, s, imidazole CH)

Mass Spectrum (EI): m/z 281 ($M^+$, as a free compound)

EXAMPLE 8

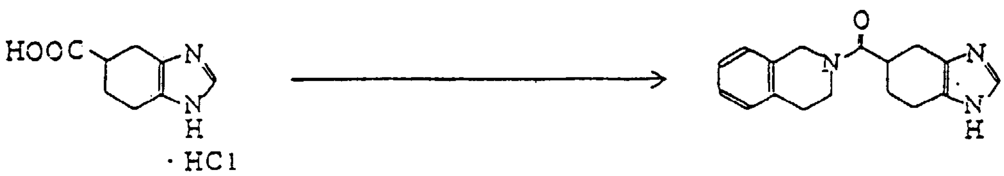

To 5 mℓ of thionyl chloride was added 0.58 g (0.98 mmol) of 4,5,6,7-tetrahydrobenzimidazole-5-carboxylic acid hydrochloride having a purity of 34.5% (containing sodium chloride), followed by stirring at 90°C for 4 hours. After cooling, the reaction mixture was distilled under reduced pressure to remove thionyl chloride. To the residue were added 10 mℓ of dichloromethane, 0.20 mℓ (1.57 mmol) of 1,2,3,4-tetrahydroisoquinoline, and 0.35 mℓ (2.53 mmol) of triethylamine, and the mixture was stirred at room temperature for 48 hours. To the reaction mixture was added 40 mℓ of dichloromethane, and the mixture was washed with 1N sodium hydroxide aqueous solution and dried over anhydrous magnesium sulfate. The solvent was removed from the residue by distillation under reduced pressure. The residue was subjected to silica gel column chromatography using dichloromethane/methanol/aqueous ammonia (10:1:0.1 by volume) as an eluent to obtain 0.15 g of a white foam substance, which was then recrystallized from diethyl ether/ethyl acetate to obtain 40 mg (14.8%) of 2-[(4,5,6,7-tetrahydrobenzimidazol-5-yl)carbonyl]-1,2,3,4-tetrahydroisoquinoline.

Physicochemical Properties:

Melting Point: 128-130°C

NMR (DMSO-$d_6$) δ ppm: 2.00-3.00 (7H, m, $CH_2$x3, CH, benzimidazole), 3.00 (2H, t, J=5Hz, $CH_2$), 3.40 (2H, t, J=5Hz, -$CH_2$-), 4.24 (2H, s, $CH_2N$), 7.22 (6H, m, ArH, NH)

Mass Spectrum (EI): m/z 281 ($M^+$)

EXAMPLE 9

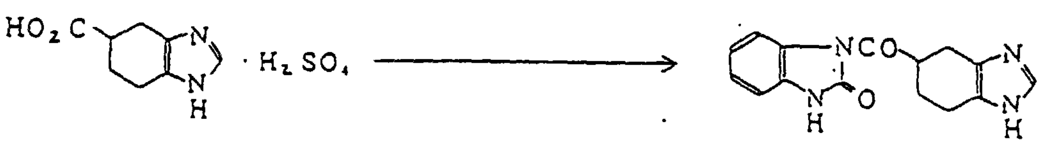

A mixture of 0.78 g of 4,5,6,7-tetrahydrobenzimidazole-5-carboxylic acid sulfate and 3 mℓ of thionyl chloride was heated at 50°C for 20 minutes, and excess thionyl chloride was removed by concentration under reduced pressure to obtain a carboxylic acid chloride. A solution of the resulting carboxylic acid chloride in 3 mℓ of dimethylformamide was added to a dimethylformamide solution (30 mℓ) of 1.61 g of 2-hydroxybenzimidazole and 0.50 g of 60% oily sodium hydride under ice-cooling, and the reaction mixture was stirred at room temperature for 1 hour, followed by concentration under reduced pressure. The residue was made acidic with 0.5N hydrochloric acid, and any insoluble matter was removed by filtration. The filtrate was made basic with potassium carbonate, and the thus formed crystals were collected by

filtration, washed with water, and stirred in acetone overnight. The resulting crystal were collected by filtration to yield 0.20 g (24%) of 1-[(4,5,6,7-tetrahydrobenzimidazol-5-yl)carbonyl]-2,3-dihydrobenzimidazol-2-one.

Physicochemical Properties:

Melting Point: 271-274°C (dec.)

| Elemental Analysis for $C_{15}H_{14}N_4O_2.0.4H_2O$: | | | |
|---|---|---|---|
| Calcd. (%): | C 62.23; | H 5.15; | N 19.35 |
| Found (%): | C 62.41; | H 5.02; | N 19.06 |

Mass Spectrum (EI): m/z 282 ($M^+$)

In the same manner as in Example 9, the following compounds were synthesized.

EXAMPLE 10

5-Methoxy-1-[(4,5,6,7-tetrahydrobenzimidazol-5-yl)-carbonyl]-2,3-dihydrobenzimidazol-2-one fumarate

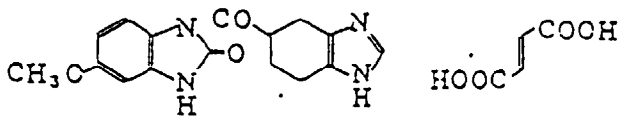

Physicochemical Properties:

Melting Point: 215-218°C (dec.) (recrystallized from methanol)

Mass Spectrum (EI): m/z 312 ($M^+$, as a free compound)

NMR (DMSO-$d_6$) δ ppm: 1.57-2.34 (2H, m), 2.34-3.10 (4H, m), 3.76 (3H, s), 3.90-4.28 (1H, m), 6.58 (2H, s), 6.32-6.84 (2H, m), 7.62 (1H, s), 7.89 (1H, d, J=8Hz)

EXAMPLE 11

1-Methyl-3-[(4,5,6,7-tetrahydrobenzimidazol-5-yl)-carbonyl]-2,3-dihydrobenzimidazol-2-one fumarate

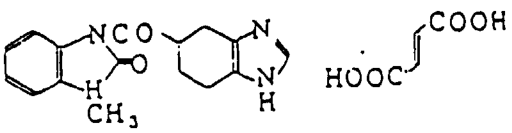

Physicochemical Properties:

Melting Point: 145-147°C (recrystallized from methanol/acetonitrile)

Mass Spectrum (EI): m/z 296 ($M^+$, as a free compound)

| Elemental Analysis for $C_{16}H_{16}N_4O_2.C_4H_4O_4.0.5H_2O$): | | | |
|---|---|---|---|
| Calcd. (%): | C 57.00; | H 5.02; | N 13.30 |
| Found (%): | C 56.91; | H 5.06; | N 13.31 |

EXAMPLE 12

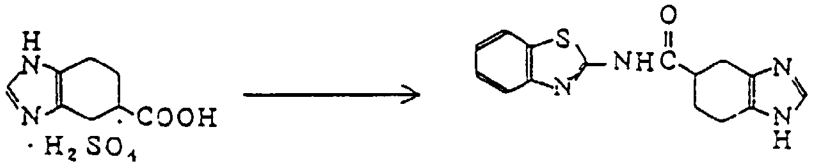

In 10 mℓ of 1,2-dichloroethane were heat-refluxed 1.32 g of 4,5,6,7-tetrahydrobenzimidazole-5-carboxylic acid sulfate and 1.78 g of thionyl chloride for 30 minutes. The excess of thionyl chloride and the solvent were removed by distillation under reduced pressure, and the residue was dissolved in 4.0 mℓ of dry dimethylformamide. The solution was added to a solution of 2.7 g of 2-aminobenzothiazole in 10 mℓ of dry dimethylformamide under ice-cooling, followed by stirring at room temperature for 1 hour. The solvent was removed by distillation under reduced pressure, and the residue was purified by silica gel column chromatography using a methylene chloride/methanol mixed solvent as a developing solvent followed by recrystallization from ethanol to obtain 0.8 g (53.7%) of N-(2-benzothiazolyl)-4,5,6,7-tetrahydrobenzimidazol-5-ylcarboxamide.

Physicochemical Properties:

Melting Point: 165-167°C

| Elemental Analysis for $C_{15}H_{14}N_4OS.0.25H_2O$: | | | | |
|---|---|---|---|---|
| Calcd. (%): | C 59.49; | H 4.82; | N 18.50; | S 10.59 |
| Found (%): | C 59.30; | H 4.73; | N 18.49; | S 10.68 |

Mass Spectrum (EI): m/z 298 ($M^+$)

In the same manner as in Example 12, the following compounds were synthesized.

EXAMPLE 13

N-(2-Benzimidazolyl)-4,5,6,7-tetrahydro-benzimidazol-5-ylcarboxamide

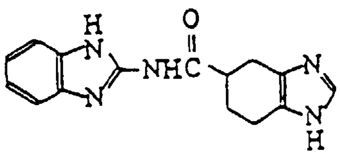

Physicochemical Properties:

Melting Point: 182-185°C

Elemental Analysis for $C_{15}H_{15}N_5O.0.6H_2O$:

Calcd. (%): C 61.67; H 5.58; N 23.97

Found (%): C 61.63; H 5.44; N 23.97

Mass Spectrum (EI): m/z 281 ($M^+$)

EXAMPLE 14

N-(Quinolin-3-yl)-4,5,6,7-tetra-hydrobenzimidazol-5-ylcarboxamide

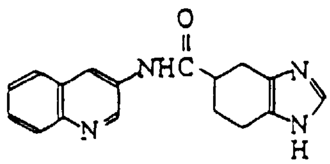

Physicochemical Properties:

Melting Point: 296-297°C

| Elemental Analysis for $C_{17}H_{16}N_4O.0.25H_2O$: | | | |
|---|---|---|---|
| Calcd. (%): | C 68.79; | H 5.60; | N 18.87 |
| Found (%): | C 68.69; | H 5.66; | N 18.75 |

Mass Spectrum (EI): m/z 292 ($M^+$)

EXAMPLE 15

N-(5-Methyl-1,3,4-thiadiazol-2-yl)-4,5,6,7-tetrahydrobenzimidazole-5-carboxamide

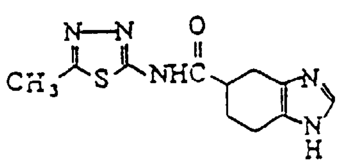

Physicochemical Properties:

Melting Point: >300°C

| Elemental Analysis for $C_{11}H_{13}N_5OS.0.2H_2H$: | | | | |
|---|---|---|---|---|
| Calcd. (%): | C 49.50; | H 5.06; | N 26.24; | S 12.01 |
| Found (%): | C 49.86; | H 4.97; | N 26.40; | S 11.68 |

Mass Spectrum (EI): m/z 263 ($M^+$)

EXAMPLE 16

N-(9-Ethylcarbazol-3-yl)-4,5,6,7-tetrahydrobenzimidazol-5-carboxamide

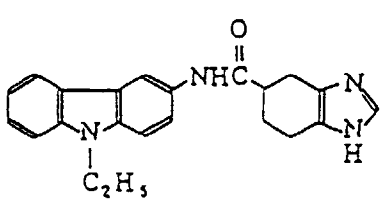

Physicochemical Properties:

Melting Point: 168-170°C

| Elemental Analysis for $C_{22}H_{22}N_4O.0.5H_2O$: | | | |
|---|---|---|---|
| Calcd. (%): | C 71.91; | H 6.31; | N 15.25 |
| Found (%): | C 71.77; | H 6.13; | N 15.13 |

Mass Spectrum (EI): m/z 358 ($M^+$)

EXAMPLE 17

N-[(4,5,6,7-Tetrahydrobenzimidazol-5-yl)carbonyl]phenothiazine hydrochloride

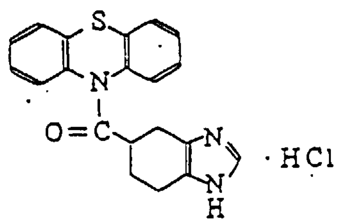

Physicochemical Properties:

Melting Point: 268-270°C

| Elemental Analysis for $C_{20}H_{17}N_3OS.HC\ell.0.5H_2O$: | | | | |
|---|---|---|---|---|
| Calcd. (%): | C 61.14; | H 4.87; | N 10.69; | Cℓ 9.02 |
| Found (%): | C 61.15; | H 4.64; | N 10.60; | Cℓ 8.59 |

Mass Spectrum (EI): m/z 347 ($M^+$, as a free compound)

EXAMPLE 18

N-(5,6-Dihydro-4H-cyclopentathiazol-2-yl)-4,5,6,7-tetrahydrobenzimidazole-5-carboxamide

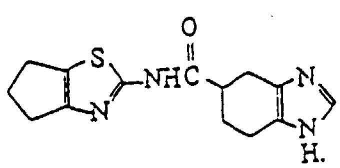

Physicochemical Properties:

| | |
|---|---|
| Melting Point: | 164-165°C |
| NMR ($DMSO_6$) δ ppm: | 1.70-3.00 (13H), 7.426 (1H) |
| Mass Spectrum (EI): | m/z 288 ($M^+$), 255 |

EXAMPLE 19

N-(Pyrimidin-2-yl)-4,5,6,7-tetrahydro-benzimidazol-5-carboxamide dihydrochloride

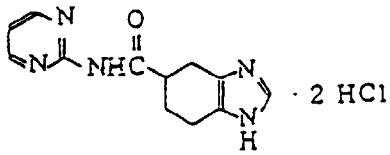

Physicochemical Properties:

Melting Point: 287-289°C

| Elemental Analysis for $C_{12}H_{13}N_5O.2HC\ell.1.4H_2O$: | | | | |
|---|---|---|---|---|
| Calcd. (%): | C 42.22; | H 5.25; | N 20.51; | Cℓ 20.77 |
| Found (%): | C 42.35; | H 5.00; | N 20.69; | Cℓ 20.45 |

Mass Spectrum (EI): m/z 243 ($M^+$, as a free compound)

EXAMPLE 20

N-(Pyridin-3-yl)-4,5,6,7-tetrahydro-benzimidazole-5-carboxamide dihydrochloride

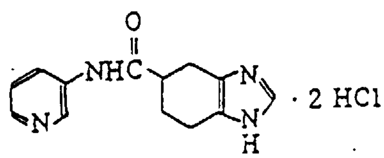

Physicochemical Properties:

Melting Point: 285-287°C

| Elemental Analysis for $C_{13}H_{14}N_4O.2HC\ell$: | | | |
|---|---|---|---|
| Calcd. (%): | C 49.54; | H 5.12; | N 17.77 |
| Found (%): | C 49.74; | H 5.26; | N 17.53 |

Mass Spectrum (EI): m/z 242 ($M^+$, as a free compound)

EXAMPLE 21

N-(3-Ethoxycarbonyl-4,5,6,7-tetrahydrobenzo[b]thiophen-2-yl)-4,5,6,7-tetrahydrobenzimidazole-5-carboxamide

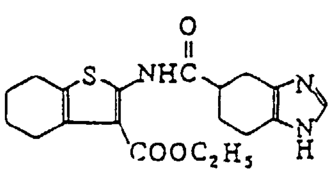

Physicochemical Properties:

Melting Point: 159-161°C

| Elemental Analysis for $C_{19}H_{23}N_3O_3S$: | | | | |
|---|---|---|---|---|
| Calcd. (%): | C 61.10; | H 6.21; | N 11.25; | S 8.59 |
| Found (%): | C 60.87; | H 6.16; | N 11.05; | S 8.62 |

Mass Spectrum (EI): m/z 373 ($M^+$)

EXAMPLE 22

N-(Indazol-6-yl)-4,5,6,7-tetrahydrobenzimidazole-5-carboxamide

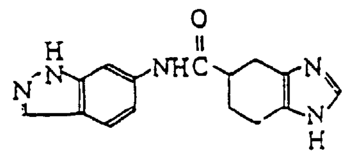

Physicochemical Properties:

Melting Point: >300°C

| Elemental Analysis for $C_{15}H_{15}N_5O$: | | | |
|---|---|---|---|
| Calcd. (%): | C 64.04; | H 5.37; | N 24.89 |
| Found (%): | C 63.79; | H 5.42; | N 24.75 |

Mass Spectrum (EI): m/z 281 ($M^+$)

EXAMPLE 23

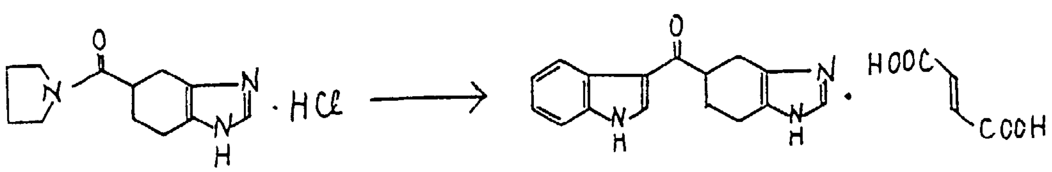

4 g of N-[(4,5,6,7-tetrahydrobenzimidazol-5-yl)carbonyl]pyrrolidine hydrochloride obtained in Example 5 were added to 40 mℓ of dichloroethane, and 2.74 g of indole and 4.4 mℓ of phosphorus oxychloride were added thereto. The mixture was stirred at 80 to 85°C for 7 hours and then at room temperature overnight. To the mixture was added 40 mℓ of a cold potassium carbonate aqueous solution, followed by extraction with chloroform. The extract was dried over anhydrous magnesium sulfate, and the solvent was removed by distillation under reduced pressure. The residue was subjected to column chromatography using chloroform/methanol as an eluent to obtain 1.82 g of 5-[(indol-3-yl)carbonyl] 4,5,6,7-tetrahydrobenzimidazole as a foam substance. In 1 mℓ of methanol were dissolved 0.16 g of the resulting product and 0.06 g of fumaric acid, and 5 mℓ of acetonitrile was added to the solution. The formed crystals were collected by filtration to obtain 0.13 g of 5-[(indol-3-yl)carbonyl]-4,5,6,7-tetrahydrobenzimidazole fumarate.

Physicochemical Properties:

Melting Point: 153-154°C

| Elemental Analysis for $C_{16}H_{15}N_3O.C_4H_4O_4.0.15CH_3CN.0.65H_2O$: | | | |
|---|---|---|---|
| Calcd. (%): | C 61.07; | H 5.24; | N 11.05 |
| Found (%): | C 61.11; | H 5.01; | N 11.04 |

Mass Spectrum (FAB): m/z 266 ($M^{+}$+1, as a free compound)

In the same manner as in Example 23, the following compounds were synthesized.

EXAMPLE 24

5-[(1,2-Dimethylindol-3-yl)carbonyl]-4,5,6,7-tetrahydrobenzimidazole·3/4 fumarate

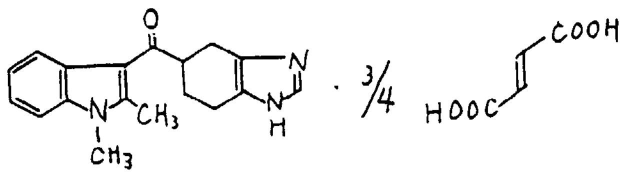

Physicochemical Properties:

Melting Point: 220-223°C

Elemental Analysis for $C_{18}H_{19}N_3O.3/4C_4H_4O_4$:

Calcd. (%): C 66.30; H 5.83; N 11.05

Found (%): C 66.50; H 5.83; N 11.13

Mass Spectrum (EI): m/z 293 ($M^{+}$, as a free compound)

EXAMPLE 25

5-[(2-Methylindol-3-yl)carbonyl]-4,5,6,7-tetrahydrobenzimidazole·½fumarate

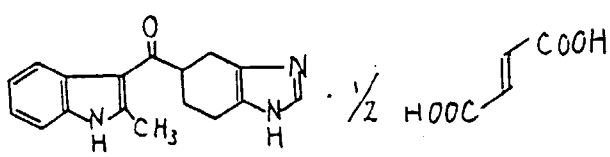

Physicochemical Properties:

Melting Point: 221-223°C

| Elemental Analysis for $C_{17}H_{17}N_3O$. ½$C_4H_4O_4$.0.25$H_2O$: | | | |
|---|---|---|---|
| Calcd. (%): | C 66.75; | H 5.75; | N 12.29 |
| Found (%): | C 66.73; | H 5.75; | N 12.29 |

Mass Spectrum (EI): m/z 279 ($M^{+}$, as a free compound)

EXAMPLE 26

5-[(2-Benzylindol-3-yl)-carbonyl]-4,5,6,7-tetrahydrobenzimidazole·fumarate

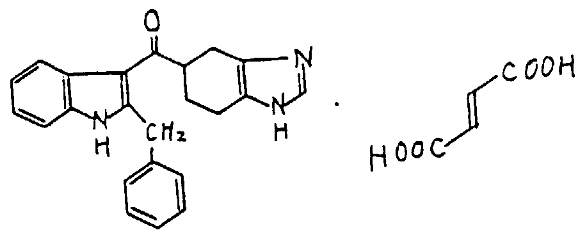

Physicochemical Properties:

Melting Point: 183-186°C

| Elemental Analysis for $C_{23}H_{21}N_3O.C_4H_4O_4.0.1H_2O$: | | | |
|---|---|---|---|
| Calcd. (%): | C 68.52; | H 5.37; | N 8.88 |
| Found (%): | C 68.38; | H 5.50; | N 8.87 |

Mass Spectrum (EI): m/z 355 ($M^+$, as a free compound)

EXAMPLE 27

5-[(5-Methoxyindol-3-yl)carbonyl]-4,5,6,7-tetrahydrobenzimidazole·3/4 fumarate

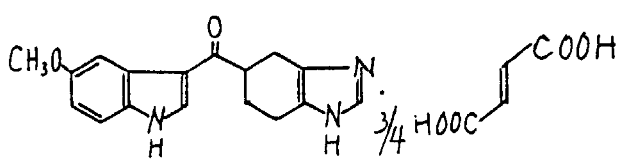

Physicochemical Properties:

Melting Point: 162-164°C

| Elemental Analysis for $C_{17}H_{17}N_3O_2.3/4C_4H_4O_4.0.2CH_3CN.0.85H_2O$: | | | |
|---|---|---|---|
| Calcd. (%): | C 60.36; | H 5.54; | N 11.04 |
| Found (%): | C 60.33; | H 5.25; | N 10.93 |

Mass Spectrum (EI): m/z 295 ($M^+$, as a free compound)

EXAMPLE 28

5-[(5-Chloro-2-methylindol-3-yl)-carbonyl]-4,5,6,7-tetrahydrobenzimidazole·fumarate

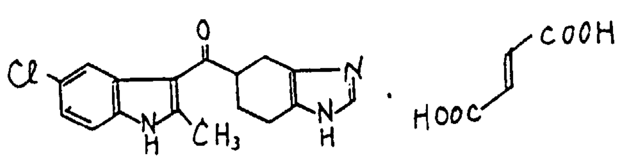

Physicochemical Properties:

Melting Point: 212-213°C

| Elemental Analysis for $C_{17}H_{16}N_3OC\ell.C_4H_4O_4$: | | | | |
|---|---|---|---|---|
| Calcd. (%): | C 58.68; | H 4.67; | N 9.78; | Cℓ 8.25 |
| Found (%): | C 58.43; | H 4.91; | N 9.67; | Cℓ 8.24 |

Mass Spectrum (FAB, Pos): m/z 314 ($M^{+}$+1, as a free compound)

EXAMPLE 29

5-[(5-Nitroindol-3-yl)carbonyl-4,5,6,7-tetrahydrobenzimidazole

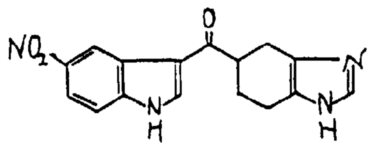

Physicochemical Properties:

Melting Point: 227-229°C
NMR (DMSO-$d_6$) 100M, δ: 2.00 (2H, m), 2.70 (4H, m), 3.63 (1H, m), 7.44 (1H, s), 7.64 (1H, d, $J_{6.7}$=12Hz), 8.10 (1H, dd, $J_{6.7}$=12Hz, $J_{4.6}$=4Hz), 8.72 (1H, s), 9.07 (1H, d, $J_{4.6}$=4Hz), 12.56 (1H, br)
Mass Spectrum (EI): m/z 310 ($M^{+}$)

EXAMPLE 30

5-[(5-Methoxycarbonylindol-3-yl)-carbonyl]-4,5,6,7-tetrahydrobenzimidazole

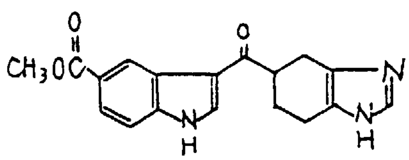

Physicochemical Properties:

Melting Point: 205-209°C
NMR (DMSO-$d_6$) 100M δ: 1.90-2.15 (2H, m), 2.83 (4H, br), 3.75 (1H, br), 7.56 (1H, d, $J_{6.7}$=12Hz), 7.84 (1H, dd, $J_{6.7}$=12Hz, $J_{4.6}$=3Hz), 8.62 (1H, d, $J_{2.NH}$=4Hz), 8.90 (2H, s), 12.60 (1H, d, $J_{2.NH}$=4Hz)
Mass Spectrum (EI): m/z 323 ($M^{+}$)

EXAMPLE 31

5-[(5-Hydroxyindol-3-yl)carbonyl]-4,5,6,7-tetrahydrobenzimidazole·½fumarate

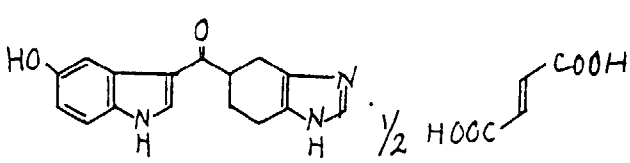

Physicochemical Properties:

| | |
|---|---|
| Melting Point: | 282-286°C |
| NMR (DMSO-$d_6$) 100M δ: | 1.90 (2H, br), 2.85 (4H, br), 3.74 (1H, br), 6.76 (1H, s), 6.84 (1H, dd, $J_{6.7}$=12Hz, $J_{4.6}$=4Hz), 7.41 (1H, d, $J_{6.7}$=12Hz), 7.74 (1H, d, $J_{4.6}$=4Hz), 8.50 (1H, d, $J_{2.NH}$=5Hz), 9.07 (1H, s), 11.95 (1H, d, $J_{2.NH}$) |
| Mass Spectrum (EI): | m/z 281 ($M^+$, as a free compound) |

EXAMPLE 32

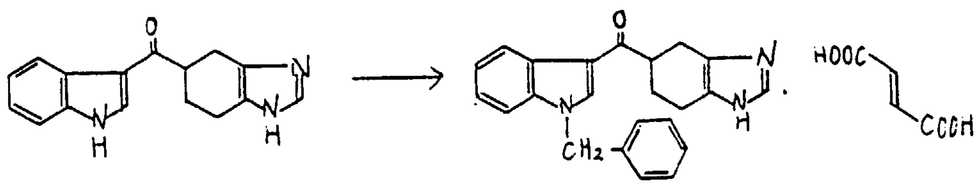

To 5 mℓ of dry dimethylformamide was added 0.04 g of 60% oily sodium hydride, and 0.51 g of 5-[(indol-3-yl) carbonyl]-4,5,6,7-tetrahydro-1H-benzimidazole as obtained in Example 23 was slowly added thereto at room temperature. Thirty minutes later, 0.07 g of benzyl bromide was slowly added thereto at 0°C, followed by stirring at room temperature overnight. To the reaction mixture were added 20 mℓ of water and 20 mℓ of chloroform for liquid-liquid separation. The organic layer was washed with water and dried over anhydrous magnesium sulfate. The solvent was removed by distillation, and the residue was subjected to chromatography using chloroform/methanol as an eluent. The resulting foam substance (0.12 g) was recrystallized together with 0.04 g of fumaric acid from ethanol/ethyl acetate to obtain 0.10 g of 5-[1-benzylindol-3-yl)carbonyl]-4,5,6,7-tetrahydrobenzimidazole fumarate.

Physicochemical Properties:

Melting Point: 117-118°C

| Elemental Analysis for $C_{23}H_{21}N_3O.C_4H_4O_4.0.75H_2O$: | | | |
|---|---|---|---|
| Calcd. (%): | C 66.86; | H 5.51; | N 8.66 |
| Found (%): | C 66.83; | H 5.48; | N 8.88 |

Mass Spectrum (EI): m/z 321 ($M^+$, as a free compound)

In the same manner as in Example 32, the following compounds were synthesized.

EXAMPLE 33

5-[(1-Cyclohexylmethylindol-3-yl)carbonyl]-4,5,6,7-tetrahydrobenzimidazole·fumarate

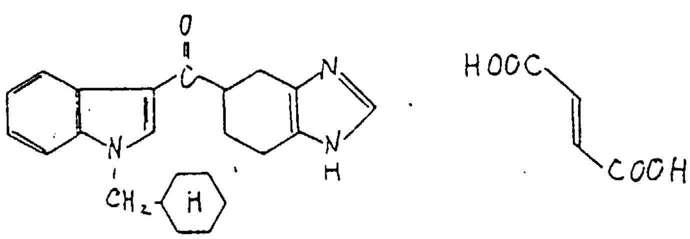

Physicochemical Properties:

Melting Point: 95-100°C (ethanol/ethyl acetate)

| Elemental Analysis for $C_{23}H_{27}N_3O.C_4H_4O_4.0.5AcOEt$ : | | | |
|---|---|---|---|
| Calcd. (%): | C 62.46; | H 7.05; | N 7.54 |
| Found (%): | C 62.59; | H 6.69; | N 7.19 |

Mass Spectrum (EI): m/z; 361 ($M^+$, as a free compound)

EXAMPLE 34

5-[(1-Allylindol-3-yl)carbonyl]-4,5,6,7-tetrahydrobenzimidazole·fumarate

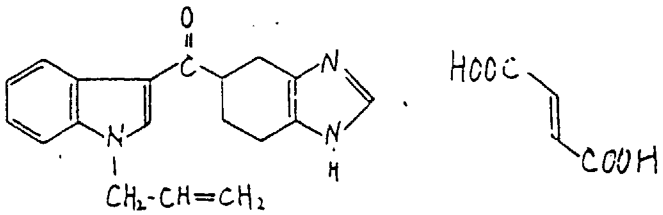

Physicochemical Properties:

Melting Point: 144-145°C (methanol/ethyl acetate)

| Elemental Analysis for $C_{19}H_{19}N_3O.C_4H_4O_4.0.35AcOEt.0.3H_2O$: | | | |
|---|---|---|---|
| Cacld. (%): | C 64.03; | H 5.81; | N 9.18 |
| Found (%): | C 64.00; | H 5.74; | N 9.17 |

Mass Spectrum (EI): m/z 305 ($M^+$, as a free compound)

EXAMPLE 35

5-[(1-n-Butylindol-3-yl)carbonyl]-4,5,6,7-tetrahydrobenzimidazole·fumarate

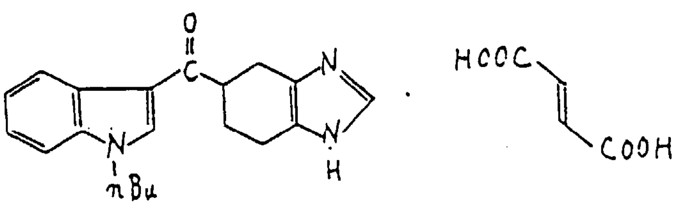

Physicochemical Properties:

Melting Point: 104-106°C (ethanol/acetonitrile)

| Elemental Analysis for $C_{20}H_{23}N_3O.C_4H_4O_4.0.8H_2O$: | | | |
|---|---|---|---|
| Calcd. (%): | C 63.78; | H 6.38; | N 9.30 |
| Found (%): | C 63.82; | H 6.14; | N 9.33 |

Mass Spectrum (EI): m/z 321 ($M^+$, as a free compound)

EXAMPLE 36

5-[[1-(2-Propynyl)indol-3-yl]carbonyl]-4,5,6,7-tetrahydrobenzimidazole·fumarate

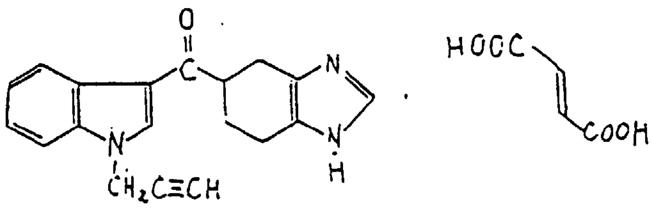

Physicochemical Properties:

Melting Point: 130-131°C (ethanol/acetonitrile)

| Elemental Analysis for $C_{19}H_{17}N_3O.C_4H_4O_4.1.3H_2O$: | | | |
|---|---|---|---|
| Calcd. (%): | C 62.38; | H 5.37; | N 9.49 |
| Found (%): | C 62.38; | H 5.19; | N 9.21 |

Mass Spectrum (EI): m/z 303 ($M^+$, as a free compound)

EXAMPLE 37

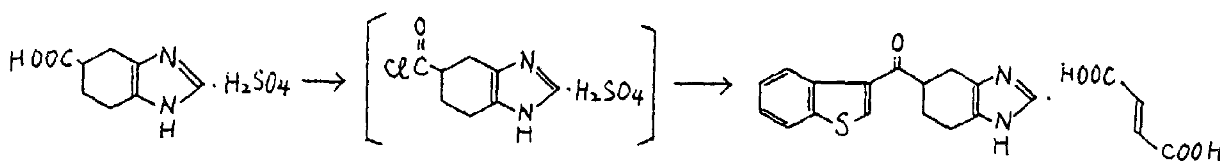

In 5 mℓ of acetonitrile was suspended 0.53 g of 4,5,6,7-tetrahydrobenzimidazole-5-carboxylic acid sulfate, and 0.29 mℓ of thionyl chloride was added to the suspension. The suspension was stirred at 55 to 60°C for 1 hour, and the solvent was removed by distillation under reduced pressure. To the residue was added 4.6 mℓ of benzothiophene, and 0.4 g of aluminum chloride was then added thereto. After stirring at 60°C for 3 hours, the reaction mixture was poured into a cold potassium carbonate aqueous solution. The solution was adjusted to a pH of 8 to 9 and extracted with chloroform. The organic layer was dried over anhydrous magnesium sulfate, and the solvent was removed by distillation. The residue was purified by silica gel column chromatography using chloroform/methanol as an eluent to obtain 5-[(benzothiophen-3-yl)carbonyl]-4,5,6,7-tetrahydrobenzimidazole. The product was treated with an equimolar amount of fumaric acid in usual manner and recrystallized from ethanol/acetonitrile to obtain 0.04 g of 5-[(benzothiophen-3-yl) carbonyl]-4,5,6,7-tetrahydrobenzimidazole fumarate.

Physicochemical Properties:

Melting Point: 135-137°C

| Elemental Analysis for $C_{16}H_{14}N_2OS.C_4H_4O_4.0.3EtOH.0.2H_2O$): | | | | |
|---|---|---|---|---|
| Cacld. (%): | C 59.50; | H 4.90; | N 6.74; | S 7.71 |
| Found (%): | C 59.41; | H 5.07; | N 6.53; | S 7.91 |

Mass Spectrum (EI): m/z 282 ($M^+$, as a free compound)

EXAMPLE 38

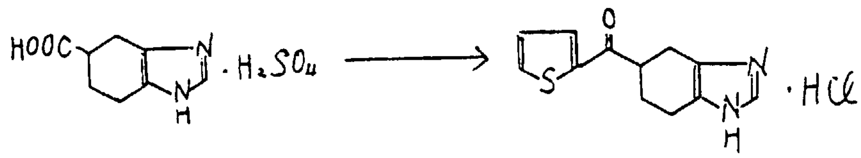

A mixture of 2 g of polyphosphoric acid, 5 mℓ of thiophene, and 2.91 g of 4,5,6,7-tetrahydrobenzimidazole-5-carboxylic acid sulfate was stirred at 100°C for 8 hours. After cooling, 20 mℓ of cold water was added thereto, and the reaction mixture was washed with toluene (20 mℓ x 2). The aqueous layer was adjusted to a pH of 8 to 9 with potassium carbonate and extracted from chloroform. The organic layer was dried over anhydrous magnesium sulfate, and the solvent was removed by distillation. The residue was treated with a 4N solution of hydrogen chloride in ethyl acetate and then recrystallized from methanol/acetonitrile to obtain 0.12 g of 5-[(thiophen-2-yl)carbonyl]-4,5,6,7-tetrahydrobenzimidazole hydrochloride.

Physicochemical Properties:

Melting Point: 218-220°C

| Elemental Analysis for $C_{12}H_{12}N_2OS.HC\ell$: | | | | |
|---|---|---|---|---|
| Calcd. (%): | C 53.63; | H 4.88; | N 10.42; | S 11.93 |
| Found (%): | C 53.25; | H 4.98; | N 10.62; | S 11.70 |

Mass Spectrum (EI): m/z; 232 ($M^+$, as a free compound)

EXAMPLE 39

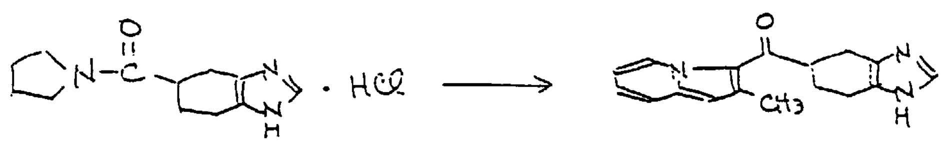

To a solution of 0.50 g of N-[(4,5,6,7-tetrahydrobenzimidazol-5-yl)carbonyl]pyrrolidinehydrochloride and 0.39 g of 2-methylindolizine in 5 mℓ of 1,2-dichloroethane was added dropwise 0.90 g of phosphorus oxychloride. The reaction mixture was refluxed at 85°C for one night. After cooling to room temperature, 5 mℓ of water was added thereto. The organic layer was removed, and 10 mℓ of chloroform was added to the aqueous layer. The solution was adjusted to a pH of 9 with a 20% aqueous solution of sodium hydroxide and then extracted with chloroform. The organic layer was dried over anhydrous magnesium sulfate, and the solvent was removed by distillation. The residue was purified by silica gel column chromatography using chloroform/methanol as an eluent, followed by recrystallization from ethanol to obtain 0.21 g of 5-[(2-methylindolizin-3-yl)carbonyl]-4,5,6,7-tetrahydrobenzimidazole.

Physicochemical Properties:

Melting Point: 260-264°C

| Elemental Analysis for $C_{17}H_{17}N_3O.0.15C_2H_5OH.0.2H_2O$: | | | |
|---|---|---|---|
| Calcd. (%): | C 71.68; | H 6.36; | N 14.50 |
| Found (%): | C 71.71; | H 6.16; | N 14.46 |

Mass Spectrum (EI): m/z 279 ($M^+$)

EXAMPLE 40

In the same manner as in Example 39, except for replacing 2-methylindolizine with pyrrole, 5-[(2-pyrrolyl)carbonyl]-4,5,6,7-tetrahydrobenzimidazole of formula shown below was synthesized.

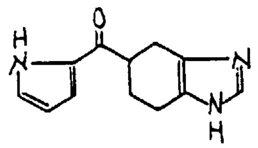

Physicochemical Properties:

Melting Point: 225 - 226°C

| Elemental Analysis for $C_{12}H_{13}N_3O$: | | | |
|---|---|---|---|
| Calcd. (%): | C 66.96; | H 6.09; | N 19.52 |
| Found (%): | C 66.74; | H 6.23; | N 19.41 |

Mass Spectrum (EI): m/z 215 ($M^+$)

EXAMPLE 41

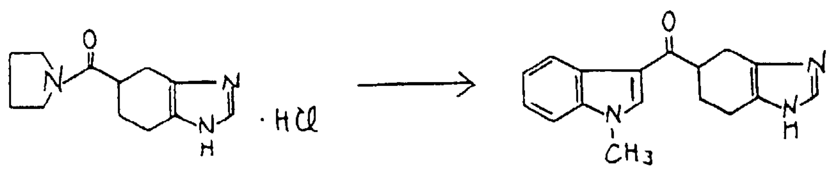

To a suspension of 7.0 g of N-[(4,5,6,7-tetrahydrobenzimidazol-5-yl)carbonyl]pyrrolidinehydrochloride and 5.4 g of N-methylindole in 70 mℓ of ethylene chloride was added 12.6 g of phosphorus oxychloride, and the mixture was stirred at 80 to 85°C for 7 hours. After allowing the mixture to cool, the mixture was cooled to 0 to 5°C, and 70 mℓ of cold water was slowly added to the reaction mixture while maintaining the temperature of the mixture below room temperature to thereby decompose the excess of phosphorus oxychloride. The organic layer was removed, and the aqueous layer was adjusted to a pH of 9 with a 20% sodium hydroxide aqueous solution under cooling, followed by extraction with chloroform. To the chloroform layer was added 70 mℓ of water, and 6N hydrochloric acid was added thereto under ice-cooling while stirring to adjust to a pH of from 2.4 to 2 8. The chloroform layer was removed. The aqueous layer was washed with chloroform, and 40 mℓ of methanol was added thereto. Thc solution was made alkaline with a 20% sodium hydroxide aqueous solution while cooling. The formed crystals were collected by filtration and washed with a cold 1:1 (by volume) mixture of methanol and water to give 6.87 g (89.9%) of 5-[(1-methylindol-3-yl) carbonyl]-4,5,6,7-tetrahydrobenzimidazole.

Physicochemical Properties:

| | |
|---|---|
| Melting Point: | 139-141°C |
| Mass Spectrum (EI): | m/z 279 ($M^+$) |
| $^1$H-NMR (CDCℓ$_3$-DMSO-d$_6$): | 1.80-2.32 (m, 2H), 2.56-3.04 (m, 4 H), 3.32-3.60 (m, 1H), 3.90 (s, 3H), 7.12-7.20 (m, 3H), 7.40 (s, 1H), 7.92 (s, 1H), 8.20-8.40 (m, 1H) |

| Elemental Analysis for $C_{17}H_{17}N_3O.0.2EtOH.0.35H_2O$: | | | |
|---|---|---|---|
| Calcd. (%): | 70.88; | H 6.46; | N 14.25 |
| Found (%): | 70.83; | H 6.50; | N 14.23 |

EXAMPLE 42

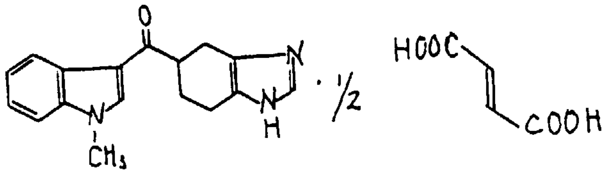

5-[(1-Methylindo1-3-yl)carbonyl]1-4,5,6,7-tetrahydrobenzimidazole was treated with a half molecular amount of fumaric acid in ethanol in known manner to obtain 5-[(1-methylindol-3-yl)carbonyl]-4,5,6,7-tetrahydrobenzimidazole ½fumarate.

Physicochemical Properties:

Melting Point: 224-225°C

Elemental Analysis for $C_{19}H_{19}N_3O_3$:

Calcd. (%): C 67.64; H 5.68; N 12.45

Found (%): C 67.56; H 5.66; N 12.35

Mass Spectrum (FAB): m/z 280 ($M^+$+1 , as a free compound)

EXAMPLE 43

Optical Resolution (1) of 5-[(1-Methylindol-3-yl carbonyl]-4,5,6,7-tetrahydrobenzimidazole

(a) To 60 mℓ of methanol was added 5.87 g of 5-[(1-methylindol-3-yl)carbonyl]-4,5,6,7-tetrahydrobenzimidazole obtained in Example 41, and a solution of 7.52 g of (+)-dibenzoyltartaric acid in 240 mℓ of methanol was added thereto to form a clear solution. On leaving the solution to stand at room temperature for one night, there were precipitated crystals, which were collected by filtration and recrystallized three times from dimethylformamide/ water to obtain 2.30 g of (R)-(-)-5-[ (1-methylindol-3-yl)carbonyl]-4,5,6,7-tetrahydrobenzimidazole (+)-dibenzoyltartrate.
Physicochemical Properties:
$[\alpha]_D^{20}$ = +30.6° (c=1.10, dimethylformamide)
Melting Point: 169.0-170.0°C

| Elemental Analysis for $C_{17}H_{17}N_3O.C_{18}H_{14}O_8.H_2O$: | | | |
|---|---|---|---|
| Calcd. (%): | C 64.12; | H 5.07; | N 6.41 |
| Found (%): | C 64.13; | H 5.03; | N 6.55 |

Mass Spectrum (FAB): m/z 280 ($M^+$ + 1, as a free compound)
(b) To a 2N hydrochloric acid aqueous solution was added 2.2 g of the compound obtained in (a) above, and the solution was washed with ethyl acetate and then adjusted to a pH of about 9 with sodium carbonate. The aqueous layer was extracted with chloroform/methanol (4:1 by volume). The extract was dried over anhydrous magnesium sulfate, and the solvent was removed by distillation to obtain 0.94 g of (R)-(-)-[(5-1-methylindol-3-yl)carbonyl]-4,5,6,7-tetrahydrobenzimidazole as a foam substance.
$[\alpha]_D^{20}$ = -16.5° (c=1.13, methanol)
(c) (R)-(-)-5-[(1-methylindol-3-yl)carbonyl]-4,5,6,7-tetrahydrobenzimidazole (0.56 g) obtained in (b) above was treated with 0.21 g of fumaric acid in methanol/acetonitrile to obtain 0.64 g of (R)-(-)-5-[(1-methylindol-3-yl)carbonyl]-4,5,6,7-tetrahydrobenzimidazole fumarate.

$[\alpha]_D^{20}$=-28.1° (C=1.22, methanol)
Melting Point: 150.5-151.5°C

| Elemental Analysis for $C_{17}H_{17}N_3O.C_4H_4O_4.0.35CH_3CN.0.25H_2O$: | | | |
|---|---|---|---|
| Calcd. (%): | C 62.91; | H 5.49; | N 11.33 |
| Found (%): | C 62.94; | H 5.41; | N 11.35 |

Mass Spectrum (EI): m/z 279 ($M^+$, as a free compound)

EXAMPLE 44

In ethanol/ethyl acetate was dissolved 100 mg of (R)-(-)-5-[ (1-methyl-3-indolyl)carbonyl]-4,5,6,7-tetrahydrobenzimidazole obtained in Example 43(b), and a solution of hydrogen chloride in ethyl acetate was added thereto. The thus formed crystals were collected and recrystallized from ethanol to obtain 70 mg of (R)-(-)-5-[(1-methylindol-3-yl)carbonyl]-4,5,6,7-tetrahydrobenzimidazole hydrochloride.
$[\alpha]_D$ = -42.9° (c=1.02, methanol)
Melting Point: 215-230°C

| Elemental Analysis for $C_{17}H_{17}N_3O.HC\ell$: | | | | |
|---|---|---|---|---|
| Calcd. (%): | C 64.66; | H 5.75; | N 13.31; | $C\ell$ 11.23 |
| Found (%): | C 64.37; | H 5.80; | N 13.12; | $C\ell$ 11.17 |

Mass Spectrum (EI): m/z 279 ($M^+$, as a free compound)

EXAMPLE 45

Optical Resolution (2) of 5-[(1-methylindol-3-yl)carbonyl]-4,5,6,7-tetrahydrobenzimidazole

(a) In the same manner as in Example 43(a), except for using (-)-dibenzoyltartaric acid, (S)-(+)-5-[(1-methylindol-3-yl)carbonyl]-4,5,6,7-tetrahydrobenzimidazole (-)-dibenzoyltartrate was obtained.
$[\alpha]_D^{20}$ = -30.3° (c=1.07, dimethylformamide)
Melting Point: 168.5-169.5°C

| Elemental Analysis for $C_{17}H_{17}N_3O.C_{18}H_{14}O_8.H_2O$: | | | |
|---|---|---|---|
| Calcd. (%): | C 64.12; | H 5.07; | N 6.41 |
| Found (%): | C 64.13; | H 5.13; | N 6.71 |

Mass Spectrum (FAB): m/z 280 ($M^+$ + 1, as a free compound)
(b) In the same manner as in Example 43(b), except for using the salt as obtained in (a) above, (S)-(+)-5-[(1-methylindol-3-yl)carbonyl]-4,5,6,7-tetrahydrobenzimidazole was obtained as a foam substance.
$[\alpha]_D^{20}$ = +16.7° (c=0.35, methanol)
(c) In the same manner as in Example 43(c), except for using (S)-(+)-5-[(1-methylindol-3-yl)carbonyl]-4,5,6,7-tetrahydrobenzimidazole as obtained in (b) above, crystals of (S)-(+)-[(1-methylindol-3-yl)carbonyl]-4,5,6,7-tetrahydrobenzimidazole fumarate were obtained.
$[\alpha]_D^{20}$ = +28.3° (c=1.14, methanol)
Melting Point: 151.0-152.0°C

| Elemental Analysis for $C_{17}H_{17}N_3O.C_4H_4O_4.0.35CH_3CN.0.25H_2O$: | | | |
|---|---|---|---|
| Calcd. (%): | C 62.91; | H 5.49; | N 11.33 |
| Found (%): | C 62.96; | H 5.39; | N 11.37 |

Mass Spectrum (EI): m/z 279 ($M^+$, as a free compound)

EXAMPLE 46

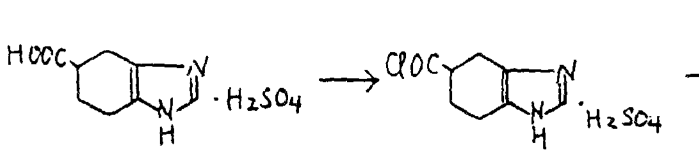

4,5,6,7-Tetrahydrobenzimidazole-5-carboxylic acid sulfate (1.32 g) was refluxed in 10 mℓ of 1,2-dichloroethane together with 1.78 g of thionyl chloride for 30 minutes, and the excess of thionyl chloride and the solvent were removed by distillation under reduced pressure. To the residue was added 10 mℓ of 1,2-dichloroethane, and 1.6 mℓ of indoline was added dropwise thereto at 30°C or lower while sitrring, followed by stirring at room temperature for 2 hours. The reaction mixture was successively extracted once with 30 mℓ of water and twice with 20 mℓ of water. The combined aqueous layer was adjusted to a pH of 9 to 10 with a 10% sodium hydroxide aqueous solution and then extracted with methylene chloride. The combined methylene chloride layer was washed with water and dried over anhydrous magnesium sulfate. The solvent was removed by distillation under reduced pressure. The residue was recrystallized from ethyl acetate to obtain 1.1 g (82.7%) of 5 [(2,3-dihydroindol-1-yl)carbonyl]-4,5,6,7-tetrahydrobenzimidazole.

| | |
|---|---|
| Melting Point: | 175-178°C |
| Mass Spectrum (EI): | m/z 267 ($M^+$) |
| $^1$H-NMR ($CDC\ell_3$-DMSO-$d_6$): | 1.80-2.36 (m, 2H), 2.48-3.12 (m, 5H), 3.24 (t, 2H), 4.20 (t, 2H), 6.84-7.30 (m, 3H), 7.50 (s, 1H), 8.20 (dd, 1H) |

| Elemental Analysis for $C_{16}H_{17}N_3O.0.25H_2O$: | | | |
|---|---|---|---|
| Calcd. (%): | C 70.70; | H 6.49; | N 15.46 |
| Found (%): | C 70.79; | H 6.37; | N 15.19 |

EXAMPLE 47

5-[(2,3-Dihydroindol-1-yl)carbonyl]-4,5,6,7-tetrahydrobenz-imidazole as obtained in Example 46 was treated with hydrochloric acid in ethanol in usual manner to obtain 5--[(2,3-dihydroindol-1-yl)carbonyl]-4,5,6,7--tetrahydrobenzimidazole hydrochloride of formula:

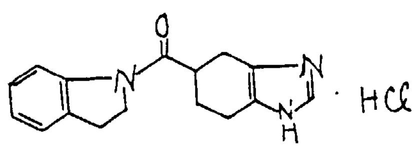

Physicochemical Properties:

Melting Point >250°C

| Elemental Analysis for $C_{16}H_{18}N_3OC\ell$: | | | | |
|---|---|---|---|---|
| Calcd. (%): | C 63.26; | H 5.97; | N 13.83; | Cℓ 11.67 |
| Found (%): | C 63.15; | H 5.97; | N 13.80; | Cℓ 11.78 |

Mass Spectrum (EI): m/z 267 ($M^+$ , as a free compound)

EXAMPLE 48

Optical Resolution (1) of 5-[(2,3-Dihydro-indol-1-yl)carbonyl]-4,5,6,7-tetrahydrobenzimidazole

(a) 4 g of 5-[(2,3-dihydroindol-1-yl)carbonyl]-4,5,6,7-tetrahydrobenzimidazole as obtained in Example 46 were dissolved in 50 mℓ of methanol, and a methanolic solution (250 mℓ) of 2.70 g of (-)-dibenzoyltartaric acid was added thereto. The formed crystals were collected by filtration, and recrystallized twice from dimethylformamide/water to obtain 2.88 g of a (-)-dibenzoyltartrate showing optical rotation of -34.0° (20°C, sodium D-line, c=0.63 g/dℓ, dimethylformamide).
Physicochemical Properties:
Melting Point: 163.5-165.0°C

| Elemental Analysis for $C_{16}H_{17}N_3O.C_{18}H_{14}O_8.0.7DMF.2.2H_2O$: | | | |
|---|---|---|---|
| Calcd. (%): | C 60.59; | H 5.66; | N 7.22 |
| Found (%): | C 60.53; | H 5.28; | N 7.26 |

Mass Spectrum (EI): m/z 267 ($M^+$ , as a free compound)
(b) The above prepared salt (2.65 g) was added to 2N hydrochloric acid, and the solution was washed with ethyl acetate. The solution was then adjusted to a pH of 9 with sodium carbonate. The aqueous layer was extracted with chloroform/methanol (4:1 by volume), and the extract was dried over anhydrous magnesium sulfate. The solvent was removed by distillation to obtain 0.95 g of a base showing optical rotation of -6.3° (20°C, sodium D-line, c=1.05 g/dℓ, methanol) as a foam substance.
Physicochemical Properties:
Melting Point: 100-106°C

| Elemental Analysis for $C_{16}H_{17}N_3O.0.2AcOEt.0.5H_2O$: | | | |
|---|---|---|---|
| Calcd. (%): | C 68.64; | H 6.72; | N 14.29 |
| Found (%): | C 68.62; | H 6.53; | N 14.30 |

Mass Spectrum (EI): m/z 267 ($M^+$)
(c) The above obtained foam base was dissolved in ethanol/ethyl acetate, and the solution was treated with a solution of hydrogen chloride in ethyl acetate to obtain 0.94 g of a crystal of a hydrochloride showing optical rotation of +19.1° (20°C, sodium D-line, c=1.06. g/dℓ, methanol).
Physicochemical Properties:
Melting Point: 241-244°C (dec.)

| Elemental Analysis for $C_{16}H_{17}N_3O.HC\ell$: | | | | |
|---|---|---|---|---|
| Calcd. (%): | C 63.26; | H 5.97; | N 13.83; | Cℓ 11.67 |
| Found (%): | C 63.18; | H 6.04; | N 13.78; | Cℓ 11.45 |

Mass Spectrum (EI): m/z 267 ($M^+$ , as a free compound)

EXAMPLE 49

Optical Resolution (2) of 5-[(2,3-Dihydroindol-1-yl)carbonyl]-4,5,6,7-tetrahydrobenzimidazole

(a) In the same manner as in Example 48(a), except for using (+)-dibenzoyltartaric acid, crystals of a (+)-dibenzoyltartrate showing optical rotation of +33.4° (20°C, sodium D-line, c=0.60, dimethylformamide) were obtained.
Physicochemical Properties:
Melting Point: 165.0-166.5°C

| Elemental Analysis for $C_{16}H_{17}N_3O.C_{18}H_{14}O_8.0.7DMF.1.85H_2O$: | | | |
|---|---|---|---|
| Calcd. (%): | C 61.13; | H 5.61; | N 7.28 |
| Found (%): | C 61.12; | H 5.28; | N 7.28 |

Mass Spectrum (EI): m/z 267 ($M^+$ , as a free compound)

(b) In the same manner as in Example 48(b), except for using the salt as obtained in (a) above, a base showing optical rotation of +7.9° (20°C, sodium D-line; c=1.06, methanol) was obtained as a foam substance.

Physicochemical Properties:

Melting Point: 98-103°C

| Elemental Analysis for $C_{16}H_{17}N_3O.0.15AcOEt.0.5H_2O$: | | | |
|---|---|---|---|
| Calcd. (%): | C 68.86; | H 6.68; | N 14.51 |
| Found (%): | C 68.65; | H 6.66; | N 14.45 |

Mass Spectrum (EI): m/z 267 ($M^+$)

(c) In the same manner as in Example 48(c), except for using the foam base as obtained in (b) above, crystals of a hydrochloride having optical rotation of -19.2° (20°C, sodium D-line, c=1.07; methanol) were obtained.

Physicochemical Properties:

Melting Point: 239-242°C (dec.)

| Elemental Analysis for $C_{16}H_{17}N_3O.HC\ell$: | | | | |
|---|---|---|---|---|
| Calcd. (%): | C 63.26; | H 5.97; | N 13.83; | Cℓ 11.67 |
| Found (%): | C 63.07; | H 5.99; | N 13.76; | Cℓ 11.58 |

Mass Spectrum (EI): m/z 267 ($M^+$ , as a free compound)

<u>EXAMPLE 50</u>

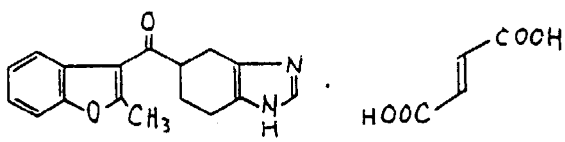

In 40 ml of acetonitrile was suspended 5.00 g of 4,5,6,7-tetrahydrobenzimidazole-5-carboxylic acid sulfate, and 2.75 ml of thionyl chloride was added to the suspension. The suspension was stirred at 55°C for 1 hour, and the solvent was distilled off under reduced pressure. To the residue was added 20 ml of nitrobenzene and 1.80 ml of 2-methylbenzofurane, and 2.20 ml of tin tetrachloride was then added thereto. After stirring at 85°C for one night, 40 ml of 1 M aqueous hydrochloric acid solution and 40 ml of ethyl ether were added thereto. The organic layer was removed, 40 ml of chloroform was added and then the solution was adjusted to a pH of 9 with 10 % aqueous solution of sodium hydroxide. The reaction solution was filtered through celite and then extracted with chloroform containing 10 % methanol. The organic layer was collected and the solvent was distilled off. To the free base obtained by subjecting the residue to silica gel column chromatography using chloroform/methanol, was added a calculated amount of fumaric acid to convert it to a fumarate; recrystallization from ethanol gave 0.14 g of 5-[(2-methylbenzofuran-3-yl)carbonyl]-4,5,6,7-tetrahydrobenz-imidazole fumarate.

Physicochemical Properties:

Melting Point 188-189°C

| Elemental Analysis for $C_{17}H_{16}N_2O_2$-$C_4H_4O_4$ | | | |
|---|---|---|---|
| Calcd. (%) | C 63.63; | H 5.09; | N 7.07 |
| Found (%) | C 63.47; | H 5.06; | N 7.01 |

Mass Spectrum (EI): m/z 280 ($M^+$, as a free compound)

EXAMPLES 51 and 52

In the same manner as in Example 39, except for replacing 2-methylindolizine with indolizine and 1-methylindolizine, 5-[(indolizin-3-yl)carbonyl]-4,5,6,7-tetrahydrobenzimidazole and 5-[(1-methylindolizin-3-yl)carbonyl]-4,5,6,7-tetrahydro-benzimidazole respectively were synthesized:

EXAMPLE 51

5-[(indolizin-3-yl)carbonyl]-4,5,6,7-tetrahydrobenzimidazole

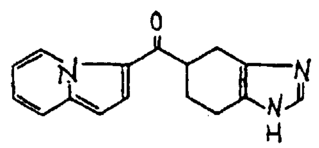

Physicochemical Properties:

Melting Point: 210-212°C

| Elemental Analysis for $C_{16}H_{15}N_3O{\cdot}0.1\ H_2O$ | | | |
|---|---|---|---|
| Calcd. (%): | C 71.94; | H 5.74; | N 15.73 |
| Found (%): | C 72.08; | H 5.79; | N 15.67 |

Mass Spectrum (EI): m/z 265 ($M^+$)

EXAMPLE 52

5-[(1-methylindolizin-3-yl) carbonyl]-4,5,6,7-tetrahydrobenzimidazole

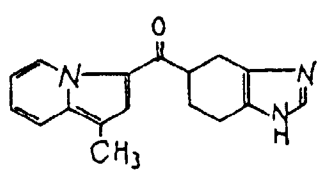

Physicochemical Properties:

Melting Point: 122-123°C

| Elemental Analysis for $C_{17}H_{17}N_3O{\cdot}0.25\ C_4H_{10}O{\cdot}0.4\ H_2O$ | | | |
|---|---|---|---|
| Calcd. (%): | C 70.87; | H 6.71; | N 13.77 |
| Found (%): | C 70.88; | H 6.68; | N 13.66 |

Mass Spectrum (EI): m/z 279 ($M^+$)

| FORMULATION EXAMPLE 1 (Tablets) | |
|---|---|
| Compound of Example 44 (hereinafter referred to Compound A) | 0.2 mg |
| Lactose | 106.4 mg |
| Corn starch | 48.0 mg |
| Hydroxypropyl cellulose | 4.8 mg |

(continued)

| FORMULATION EXAMPLE 1 (Tablets) | |
|---|---|
| Magnesium stearate | 0.6 mg |
| | Total: 160.0 mg/tablet |

Compound A (200 mg), lactose (106.4 mg), and corn starch (48 g) were uniformly mixed, and 48 mℓ of a 10% aqueous solution of hydroxypropyl cellulose was added thereto. The mixture was granulated by means of a granulator. To the granules was added 0.6 g of magnesium stearate, and the mixture was punched to obtain 1000 tablets each weighing 160 mg.

| FORMULATION EXAMPLE 2 (Powder) | |
|---|---|
| Compound A | 0.4 mg |
| Mannitol | 770.0 mg |
| Corn starch | 199.6 mg |
| Polyvinylpyrrolidone | 30.0 mg |
| | Total: 1000.0 mg |

Compound A (0.4 g), mannitol (770 g), and corn starch (199.6 g) were uniformly mixed, and 300 mℓ of a 10% aqueous solution of polyvinylpyrrolidone was added thereto, followed by granulation by means of a granulator to prepare 1 kg of powder.

| FORMULATION EXAMPLE 3 (Capsules) | |
|---|---|
| Compound A | 0.2 mg |
| Corn Starch | 198.8 mg |
| Calcium stearate | 1.0 mg |
| | Total: 200 mg |

Compound A (0.2 g), corn starch (198.8 g), and calcium stearate (1 g) were uniformly mixed, and the mixture was charged into 1000 No.3 capsules (200 mg each).

| FORMULATION EXAMPLE 4 (Syrup) | |
|---|---|
| Compound A | 0.2 mg |
| Sucrose | 8.0 mg |
| Pure water to make | 5 mℓ |

Compound A (0.2 g) and sucrose (8 g) were dissolved in distilled water to prepare 5 ℓ of a syrup.

| FORMULATION EXAMPLE 5 (Injections) | |
|---|---|
| Compound A | 0.3 mg |
| Sodium chloride | 9 mg |
| Injectable distilled water to make | 1.0 mℓ |

Compound A (300 mg) and sodium chloride (9 g) were dissolved in injectable distilled water to prepare 1000 mℓ of a solution. The solution was filtered and charged into 1000 ampoules (1 ml each) while displacing the atmosphere of the ampoule with nitrogen gas. The ampoules were sterilized by autoclaving.

## Claims

### Claims for the following Contracting States : AT, BE, CH, DE, DK, FR, GB, GR, IT, LI, LU, NL, SE

1. A tetrahydrobenzimidazole derivative of formula (I) or a pharmaceutically acceptable salt thereof:

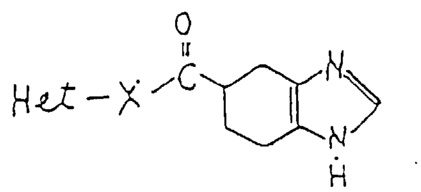

(I)

wherein Het is a heterocyclic group selected from pyrrolidine, piperidine, piperazine, morpholine, pyrrole, furan, thiophene, imidazole, oxazole, thiazole, pyrazole, isoxazole, isothiazole, triazole, thiadiazole, oxadiazole, pyridine, pyridazine, pyrimidine, pyrazine, 4H-cyclopentathiazole, indole, isoindole, 2,3-dihydroindole (indoline), isoindoline, hydroxyindole, indazole, indolizine, benzothiophene, benzofuran, benzothiazole, benzinidazole, benzoxazole, 4,5,6,7-tetrahydrobenzothiophene, 2,3-dihydrobenzimidazol-2-one, quinoline, isoquinoline, 1,2,3,4-tetrahydroquinoline, 1,2,3,4-tetrahydroisoquinoline, 1,4-benzoxazine, phenothiazine, carbazole, and β-carboline, which heterocyclic group may be substituted with 1 to 3 substituents selected from $C_1$-$C_6$ alkyl, $C_1$-$C_6$ alkenyl, $C_1$-$C_6$ alkynyl, cycloalkyl-$C_1$-$C_6$ alkyl, benzyl, phenethyl, $C_1$-$C_6$ alkoxy, nitro, hydroxyl and ($C_1$-$C_6$ alkoxy) carbonyl groups and halogen atoms; and X is a single bond or -NH- which is bonded to a carbon or nitrogen atom of the heterocyclic ring.

2. A compound as claimed in Claim 1, wherein Het is

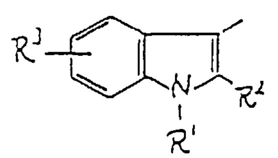

wherein $R^1$ is a hydrogen atom or a $C_1$-$C_6$ alkyl, $C_1$-$C_6$ alkenyl, $C_1$-$C_6$ alkynyl, cycloalkyl-$C_1$-$C_6$ alkyl, benzyl or phenethyl group ; $R^2$ is a hydrogen atom or a $C_1$-$C_6$ alkyl, benzyl or phenethyl group; $R^3$ is a hydrogen or halogen atom or a $C_1$-$C_6$ alkoxy, nitro, hydroxyl or ($C_1$-$C_6$ alkoxy)carbonyl group ; and X is a single bond.

3. A compound as claimed in Claim 1 or 2 wherein Het is a nitrogen-containing heterocyclic group; and X is a single bond connected to a nitrogen atom of the nitrogen-containing heterocyclic ring

4. One or more compounds according to claim 1 selected from (R)-and (S)-5-[(1-methyilndol-3-yl)carbonyl]-4,5,6,7-tetrahydrobenzimidazole,

   5-[(1-(2-propynyl)indol-3-yl]carbonyl]-4,5,6,7-tetrahydrobenzimidazole,
   5-[(2,3-dihydroindol-1-yl]carbonyl]-4,5,6,7-tetrahydrobenzimidazole,
   1-[(4,5,6,7-tetrahydobenzimidazol-5-yl)carbonyl]-2,3-dihydrobenzimidazol-2-one ;
   5-[(2-methylindolizin-3-yl)carbonyl]-4,5,6,7-tetrahydrobenzimidazole;
   3-[(indolizin-3-yl)carbonyl]-4,5,5,7-tetrahydrobenzimidazole;
   5-[(1-methylindolizin-3-yl)carbonyl]4,5,5,7-tetrahydrobenzinidazole; and pharmaceutically acceptable salts of said compounds.

5. One or more compounds according to claim 1 selected from 5-[(2-benzylindol-3-yl)-carbonyl]-4,5,6,7-tetrahydrobenzimidazole; 5-[(1-n-butylindol-3-yl)carbonyl]-4,5,6,7-tetrahydrobenzimidazole; 5-[(1-cyclohexylmethylindol-3-yl)carbonyl]-4,5,6,7-tetrahydrobenzimidazole; 5-[1-benzylindol-3-yl)carbonyl]4,5,6,7-tetrahydrobenzimidazole; N-(pyridin-3-yl)-4,5,6,7tetrahydrobenzimidazole-5-carboxamide; and N-(5,6-dihydro-4H-cyclopentathiazol-2-yl)-4,5,6,7-tetrahydrobenzimidazole-5-carboxamide; and pharmaceutically acceptable salts thereof.

**6.** A process for preparing a tetrahydrobenzimidazole derivative of formula (Ia):

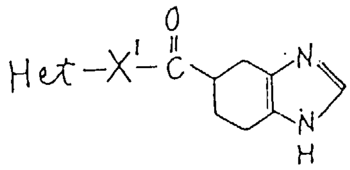

(Ia)

wherein Het is as defined in claim 1 and $X^1$ is a single bond connected to a nitrogen atom of the heterocyclic ring or -NH- connected to a carbon atom of the heterocyclic ring,
which comprises reacting amine, amide or urea (III)

$$Het\text{-}X^1\text{-}H \quad (III)$$

with carboxylic acid (II) or reactive derivative thereof

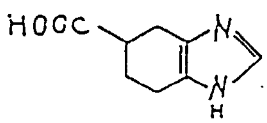

(II)

**7.** A process for preparing a tetrahydrobenzimidazole derivative of formula (Ib):

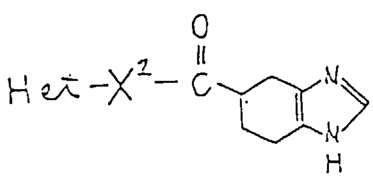

(Ib)

wherein Met is as defined in claim 1 and $X^2$ is a single bond connected to a carbon atom of the heterocyclic ring,
which comprises reacting heterocyclic compound (IIIa)

$$Het\text{-}X^2\text{-}H \quad (IIIa)$$

with carboxylic acid (II) or reactive derivative thereof

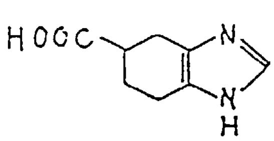

(II)

**8.** A process for preparing a tetrahydrobenzimidazole derivative of formula (Ic):

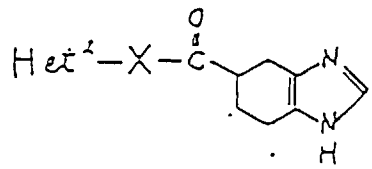

(Ic)

which comprises N-alkylating compound (Id).

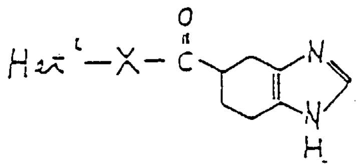

(Id)

wherein $Het^1$ is a group Het which is as defined in claim 1 and which has -NH- in its ring, X is as defined in claim 1, and $Het^2$ is $Het^1$ wherein said ring -MH- has been converted to ring

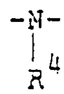

where $R^4$ is a $C_1$-$C_6$ alkyl, $C_1$-$C_6$ alkenyl, $C_1$-$C_6$ alkynyl, cycloalkyl-$C_1$-$C_6$ alkyl, benzyl or phenethyl group.

9. A process according to any of claims 6 to 8 including converting the product to salt form.

10. A pharmaceutical composition containing at least one compound according to any of claims 1 to 5 with a pharmaceutically acceptable carrier.

11. A composition according to claim 10 for use as a $5HT_3$-antagonist.

12. A composition according to claim 10 for prevention or treatment of gastrointestinal disorder; or for suppressing nausea and/or vomiting induced by chemotherapy or radiation; or for preventing or treating migraine, cluster headache or trigeminal neuralgia; or for preventing or treating anxiety or psychotic disorder.

13. A composition according to claim 10 for prevention or treatment of anaphylactic intestinal syndrome.

14. The use of a compound according to any of claims 1 to 5 for the preparation of a medicament having $5HT_3$-antagonist activity.

15. The use of a compound according to any of claims 1 to 5 for the preparation of a medicament for the prevention or treatment of gastrointestinal disorder; or for suppressing nausea and/or vomiting induced by chemotherapy or radiation; or for preventing or treating migraine, cluster headache or trigeminal neuralgia; or for preventing or treating anxiety or psychotic disorder.

16. The use of a compound according to any of claims 1 to 5 for the preparation of a medicament for the prevention or treatment of anaphylactic intestinal syndrome.

**Claims for the following Contracting State : ES**

1. A process for preparing a tetrahydrobonzimidazole derivative of formula (Ia):

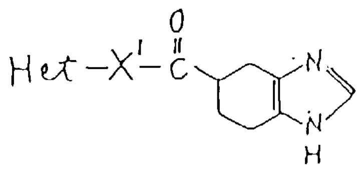

(Ia)

wherein Het is a heterocyclyc group selected from pyrrolidine, piperidine, piperazine, morpholine pyrrole, furan, thiophene, imidazole, oxazole, thiazole, pyrazole, isoxazole, isothiazole, triazole, thiadiazole, oxadiazole, pyridine, pyridazine, pyrimidine, pyrazine, 4H-cyclopentathiazole, indole, isoindole, 2,3-dihydroindole (indoline), isoindoline, hydroxyindole, indazole, indolizine, benzothiophene, benzofuran, benzothiazole, benzimidazole, benzoxazole, 4,5,6,7-tetrahydrobenzothiophene, 2,3-dihydrobenzimidazol-2-one, quinoline, isoquinoline, 1,2,3,4-tetrahydroquinoline, 1,2,3,4-tetrahydroisoquinoline, 1,4-benzoxazine, phenothiazine, carbazole, and β-carboline, which heterocyclic group may be substituted with 1 to 3 substituents selected from $C_1$-$C_6$ alkyl, $C_1$-$C_6$ alkenyl, $C_1$-$C_6$ alkynyl, cycloalkyl-$C_1$-$C_6$ alkyl, benzyl, phenethyl, $C_1$-$C_6$ alkoxy, nitro, hydroxyl ($C_1$-$C_6$ alkoxy)carbonyl groups and halogen atoms; and $X^1$ is a single bond connected to a nitrogen atom of the heterocyclic ring or -NH- connected to a carbon atom of the heterocyclic ring,
which comprises reacting amine, amide or urea (III)

$$\text{Het-}X^1\text{-H} \qquad \text{(III)}$$

with carboxylic acid (II) or reactive derivative thereof

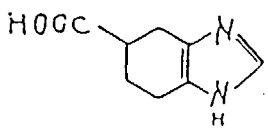

(II)

2. A process for preparing a tetrahydrobenzimidazole derivative of formula (Ib):

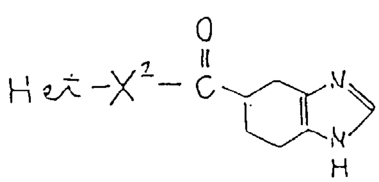

(Ib)

wherein Het is as defined in claim 1 and $X^2$ is a single bond connected to a carbon atom of the heterocyclic ring, which comprises reacting heterocyclic compound (IIIa)

$$\text{Het-}X^2\text{-H} \qquad \text{(IIIa)}$$

with carboxylic acid (II) or reactive derivative thereof

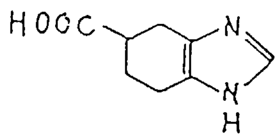

(II)

3. A process for preparing a tetrahydrobenzimidazole derivative of formula (Ic) :

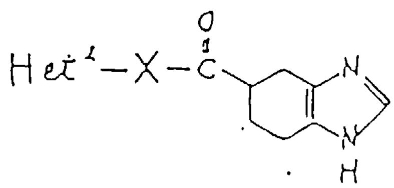

(Ic)

which comprises N-alkylating compound (Id)

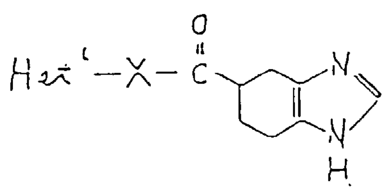

(Id)

wherein $Het^1$ is a group Het which is as defined in claim 1 and which has -NH- in its ring, X is a single bond or -NH- which is bonded to a carbon or nitrogen atom of the heterocyclic ring, and $Het^2$ is $Het^1$ wherein said ring -NH- has been converted to ring

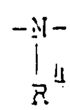

where $R^4$ is a $C_1$-$C_6$ alkyl, $C_1$-$C_6$ alkenyl, $C_1$-$C_6$ alkynyl, cycloalkyl-$C_1$-$C_6$ alkyl, benzyl or phenethyl group.

4. A method as claimed in claim 1 or 3 wherein Het is a nitrogen-containing heterocyclic group; and X is a single bond connected to a nitrogen atom of the nitrogen-containing heterocyclic ring.

5. A process as claimed in any preceding claim wherein Het is

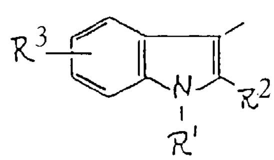

wherein $R^1$- is a hydrogen atom or a $C_1$-$C_6$ alkyl, $C_1$-$C_6$ alkenyl, $C_1$-$C_6$ alkyl, cycloalkyl-$C_1$-$C_6$ alkyl, benzyl or phenethyl group ; $R^2$ is a hydrogen atom or a $C_1$-$C_6$ alkyl, benzyl or phenethyl group; $R^1$ is a hydrogen or halogen atom or a $C_1$-$C_6$ alkoxy, nitro, hydroxyl or ($C_1$-$C_6$ alkoxy)carbonyl group; and X is a singe bond.

6. A process according to any preceding claim including converting the product to salt form.

7. A method according to claim 1, 2, 3 or 6 wherein the product compound is selected from (R)- and (5)-5-[(1-methylindol-3-yl)carbonyl)-4,5,6,7-tetrahydrobenzimidazole,

5-[(1-(2-propynyl)indol-3-yl]carbonyl]-4,5,6,7-tetrahydrobenzimidazole,
5-[(2,3-dyhydroindol-1-yl)carbonyl]-4,5,6,7-tetrahydrobenzimidazole,
1-[(4,5,6,7-tetrahydrobenzimidazol-5-yl)carbonyl]-2,3-dihydrobenzimidazol-2-one ;
5-[[2-methylindolizin-3-yl)carbonyl]-4,5,6,7-tetrahydrobenzimidazole;
3-[(indolizin-3-yl)carbonyl]-4,5,6,7-tetrahydro-benzimidazole;
5-[(1-methylindolizin-3-yl)carbonyl]4,5,6,7-tetrahydrobenzimidazole; and pharmaceutically acceptable salts of said compounds.

8. A method according to claim 1, 2, 3 or 6 wherein the product compound is selected from 5-[(2-benzylindol-3-yl) carbonyl] -4,5,6,7-tetrahydrobenzimidazole; 5-[(1-n-butylindol-3-yl)carbonyl]-4,5,6,7-tetrahydrobenzimidazole; 5-[(1-cyclohexylmethylindol-3-yl)carbonyl]-4,5,6,7-tetrahydrobenzimidazole; 5-[1-benzylindol-3-yl)carbonyl]4,5,6,7-tetrahydrobenzimidazole; N- (pyridin-3-yl)-4,5,6,7tetrahydrobenzimidazole-5-carboxamide; and N-(5,6dihydro-4H-cyclopentathiazol-2-yl)-4,5,6,7tetrahydrobenzimidazole-5-carboxamide; and pharmaceutically acceptable salts thereof.

9. A method of making a pharmaceutical composition which comprises mixing with pharmaceutically acceptable carrier at least one compound selected from tetrahydrobenzimiazole derivative of formula (I) and pharmaceutically acceptable salt thereof:

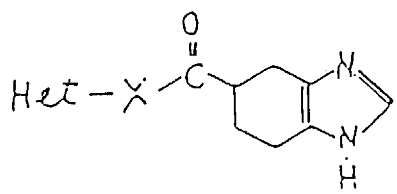

(I)

wherein Het is as defined in claim 1 and X is as defined in claim 3.

10. The use of a tetrahydroimidazole compound as defined in claim 9 for the preparation of a medicament having $5HT_3$-antagonist activity.

11. The use of a tetrahydroimidazole compound as defined in claim 9 for the preparation of a medicament for the prevention or treatment of gastrointestinal disorder; or for suppressing nausea and/or vomiting induced by chemotherapy or radiation; or for preventing or treating migraine, cluster headache or trigeminal neuralgia; or for preventing or treating anxiety or psychotic disorder.

12. The use of a tetrahydroimidazole compound as defined in claim 9 for the preparation of a medicament for the prevention or treatment of anaphylactic intestinal syndrome.

**Patentansprüche**

**Patentansprüche für folgenden Vertragsstaaten : DE, GB, FR, IT, NL, SE, CH, LI, BE, AT, LU, DK, GR**

1. Tetrahydrobenzimidazolderivat der Formel (I) oder ein pharmazeutisch annehmbares Salz davon:

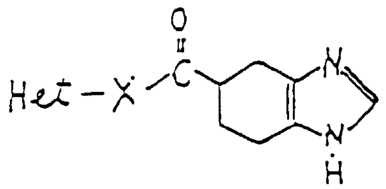

(I)

wobei Het eine heterocyclische Gruppe ist, ausgewählt aus Pyrrolidin, Piperidin, Piperazin, Morpholin, Pyrrol, Furan, Thiophen, Imidazol, Oxazol, Thiazol, Pyrazol, Isoxazol, Isothiazol, Triazol, Thiadiazol, Oxadiazol, Pyridin, Pyridazin, Pyrimidin, Pyrazin, 4H-Cyclopentathiazol, Indol, Isoindol, 2,3-Dihydroindol (Indolin), Isoindolin, Hydroxyindol, Indazol, Indolizin, Benzotiophen, Benzofuran, Benzothiazol, Benzimidazol, Benzoxazol, 4,5,6,7-Tetrahydrobenzothiophen, 2,3-Dihydrobenzimidazol-2-on, Chinolin, Isochinolin, 1,2,3,4-Tetrahydrochinolin, 1,2,3,4-Tetrahydroisochinolin, 1,4-Benzoxazin, Phenothiazin, Carbazol und β-Carbolin, wobei die heterocyclische Gruppe substituiert sein kann durch 1 bis 3 Substituenten, ausgewählt aus $C_1$-$C_6$-Alkyl-, $C_1$-$C_6$-Alkenyl-, $C_1$-$C_6$-Alkinyl-, Cycloalkyl-$C_1$-$C_6$-alkyl-, Benzyl-, Phenethyl-, $C_1$-$C_6$-ASkoxy-, Nitro-, Hydroxyl- und ($C_1$-$C_6$-Alkoxy)carbonylgruppen und Halogenatomen, und X eine Einfachbindung oder -NH-, gebunden an ein Kohlenstoff- oder Stickstoffatom des heterocyclischen Ringes, ist.

2. Verbindung nach Anspruch 1, wobei Het

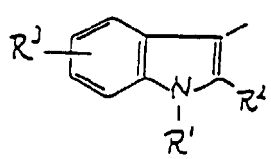

ist, wobei $R^1$ ein Wasserstoffatom oder eine $C_1$-$C_6$-Alkyl-, $C_1$-$C_6$-Alkenyl-, $C_1$-$C_6$-Alkinyl-, Cycloalkyl-$C_1$-$C_6$-alkyl-, Benzyl- oder Phenethylgruppe ist, $R^2$ ein Wasserstoffatom oder eine $C_1$-$C_6$-Alkyl-, Benzyl- oder Phenethylgruppe ist, $R^3$ ein Wasserstoff- oder Halogenatom oder eine $C_1$-$C_6$-Alkoxy-, Nitro-, Hydroxyl- oder ($C_1$-$C_6$-Alkoxy)carbonylgruppe ist und X eine Einfachbindung ist.

3. Verbindung nach Anspruch 1 oder 2, wobei Het eine stickstoffhaltige heterocyclische Gruppe ist und X eine Einfachbindung ist, die an ein Stickstoffatom des stickstoffhaltigen heterocyclischen Ringes gebunden ist.

4. Eine oder mehrere Verbindungen nach Anspruch 1, ausgewählt aus (R)- und (S)-5-[(1-Methylindol-3-yl)carbonyl]-4,5,6,7-tetrahydrobenzimidazol,

5-[[1-(2-Propynyl)indol-3-yl]carbonyl]-4,5,6,7-tetrahydrobenzimidazol,
5-[(2,3-Dihydroindol-l-yl)carbonyl]-4,5,6,7-tetrahydrobenzimidazol,
1-[(4,5,6,7-Tetrahydrobenzimidazol-5-yl)carbonyl]-2,3-dihydrobenzimidazol-2-on,
5-[(2-Methylindolizin-3-yl)carbonyl]-4,5,6,7-tetrahydrobenzimidazol,
3-[(Indolizin-3-yl)carbonyl]-4,5,6,7-tetrahydrobenzimidazol,
5-[(1-Methylindolizin-3-yl)carbonyl]4,5,6,7-tetrahydrobenzimidazol und pharmazeutisch annehmbaren Salzen dieser Verbindungen.

5. Eine oder mehrere Verbindungen nach Anspruch 1, ausgewählt aus 5-[(2-Benzylindol-3-yl)-carbonyl]-4,5,6,7-tetrahydrobenzimidazol, 5-(1-n-Butylindol-3-yl)carbonyl]-4,5,6,7-tetrahydrobenzimidazol, 5-[(1-Cyclohexylmethylindol-3-yl)carbonyl]-4,5,6,7-tetrahydrobenzimidazol, 5-[l-Benzylindol-3-yl)carbonyl]-4,5,6,7-tetrahydrobenzimidazol, N-(Pyridin-3-yl)-4,5,6,7-tetrahydrobenzimidazol-5-carboxamid und N-(5,6-Dihydro-4H-cyclopentathiazol-2-yl)-4,5,6,7-tetrahydrobenzimidazol-5-carboxamid und pharmazeutisch annehmbaren Salzen davon.

6. Verfahren zur Herstellung eines Tetrahydrobenzimidazolderivats der Formel (Ia):

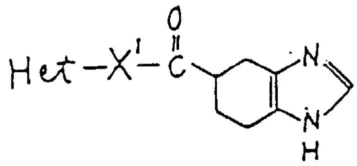

(Ia)

wobei Het, wie in Anspruch 1 definiert ist und $X^1$ eine Einfachbindung ist, die an ein Stickstoffatom des heterocyclischen Rings gebunden ist, oder -NH-, das an ein Kohlenstoffatom des heterocyclischen Ringes gebunden ist, umfassend die Umsetzung von Amin, Amid oder Harnstoff (III)

$$Het\text{-}X^1\text{-}H \qquad (III)$$

mit Carbonsäure (II) oder einem reaktionsfähigen Derivat davon

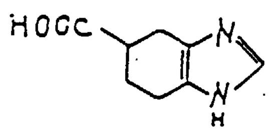

(II)

**7.** Verfahren zur Herstellung eines Tetrahydrobenzimidazolderivats der Formel (Ib):

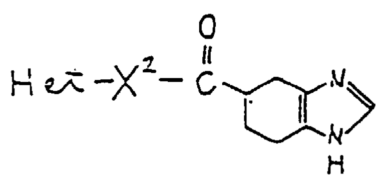

(Ib)

wobei Het wie in Anspruch 1 definiert ist und $X^2$ eine an ein Kohlenstoffatom des heterocyclischen Ringes gebundene Einfachbindung ist, umfassend die Umsetzung einer heterocyclischen Verbindung (IIIa)

$$Het\text{-}X^2\text{-}H \qquad (IIIa)$$

mit Carbonsäure (II) oder einem reaktionsfähigen Derivat davon

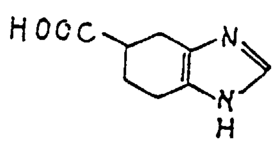

(II)

**8.** Verfahren zur Herstellung eines Tetrahydrobenzimidazolderivats der Formel (Ic):

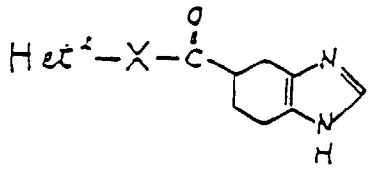

(Ic)

umfassend das N-Alkylieren einer Verbindung (Id)

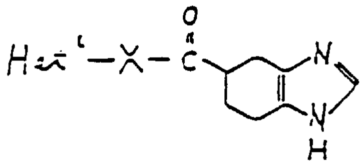

(Id)

wobei Het[1] eine Gruppe Het, wie in Anspruch 1 definiert, ist und die -NH- im Ring enthält, X wie in Anspruch 1 definiert ist und Het[2] Het[1] ist, wobei das Ring -NH- umgewandelt ist in Ring

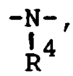

wobei $R^4$ eine $C_1$-$C_6$-Alkyl-, $C_1$-$C_6$-Alkenyl-, $C_1$-$C_6$-Alkinyl-, Cycloalkyl-$C_1$-$C_6$-alkyl- Benzyl- oder Phenethylgruppe ist.

**9.** Verfahren nach Anspruch 6 bis 8, umfassend die Umwandlung des Produktes in die Salzform.

**10.** Pharmazeutisches Mittel, enthaltend mindestens eine Verbindung nach einem der Ansprüche 1 bis 5, mit einem pharmazeutisch annehmbaren Träger.

**11.** Mittel nach Anspruch 10 zur Verwendung als $5HT_3$-Antagonist.

**12.** Mittel nach Anspruch 10 zur Verhütung oder Behandlung von Magen-Darm-Störungen oder zur Unterdrückung von Übelkeit und/oder Erbrechen, das durch Chemotherapie oder Bestrahlung hervorgerufen ist, oder zur Verhütung oder Behandlung von Migräne, Cluster headache oder Trigeminusneuralgien oder zur Verhütung oder Behandlung von Angst oder psychischen Störungen.

**13.** Mittel nach Anspruch 10 zur Verhütung oder Behandlung von anaphylaktischem Intestinalsyndrom.

**14.** Verwendung einer Verbindung nach einem der Ansprüche 1 bis 5 zur Herstellung eines Arzneimittels mit $5HT_3$-Antagonistenwirkung.

**15.** Verwendung einer Verbindung nach einem der Ansprüche 1 bis 5 zur Herstellung eines Arzneimittels zur Verhütung oder Behandlung von Magen-Darm-Störungen oder zur Unterdrückung von Übelkeit und/oder Erbrechen, das durch Chemotherapie oder Bestrahlung hervorgerufen ist oder zur Verhütung oder Behandlung von Migräne, Cluster headache oder Trigeminusneuralgien oder zur Verhütung oder Behandlung von Angst oder psychischen Störungen.

**16.** Verwendung einer Verbindung nach einem der Ansprüche 1 bis 5 zur Herstellung eines Arzneimittels zur Verhütung oder Behandlung von anaphylaktischem Intestinalsyndrom.

**Patentansprüche für folgenden Vertragsstaat : ES**

**1.** Verfahren zur Herstellung eines Tetrahydrobenzimidzaolderivats der Formel (Ia):

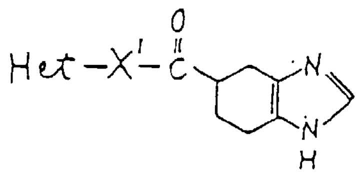

(Ia)

wobei Het eine heterocyclische Gruppe ist, ausgewählt aus Pyrrolidin, Piperidin, Piperazin, Morpholin, Pyrrol, Furan, Thiophen, Imidazol, Oxazol, Thiazol, Pyrazol, Isoxazol, Isothiazol, Triazol, Thiadiazol, Oxadiazol, Pyridin, Pyridazin, Pyrimidin, Pyrazin, 4H-Cyclopentathiazol, Indol, Isoindol, 2,3-Dihydroindol (Indolin), Isoindolin, Hydroxyindol, Indazol, Indolizin, Benzotiophen, Benzofuran, Benzothiazol, Benzimidazol, Benzoxazol, 4,5,6,7-Tetrahydrobenzothiophen, 2,3-Dihydrobenzimidazol-2-on, Chinolin, Isochinolin, 1,2,3,4-Tetrahydrochinolin, 1,2,3,4-Tetrahydroisochinolin, 1,4-Benzoxazin, Phenothiazin, Carbazol und β-Carbolin, wobei die heterocyclische Gruppe substituiert sein kann durch 1 bis 3 Substituenten, ausgewählt aus $C_1$-$C_6$-Alkyl-, $C_1$-$C_6$-Alkenyl-, $C_1$-$C_6$-Alkinyl-, Cycloalkyl-$C_1$-$C_6$-alkyl-, Benzyl-, Phenethyl-, $C_1$-$C_6$-ASkoxy-, Nitro-, Hydroxyl- und ($C_1$-$C_6$-ASkoxy)carbonylgruppen und Halogenatomen, und $X^1$ eine Einfachbindung ist, die an ein Stickstoffatom des heterocyclischen Rings gebunden ist, oder -NH-, das an ein Kohlenstoffatom des heterocyclischen Ringes gebunden ist,
umfassend die Umsetzung von Amin, Amid oder Harnstoff (III)

$$\text{Het-}X^1\text{-H} \quad \text{(III)}$$

mit Carbonsäure (II) oder einem reaktionsfähigen Derivat davon

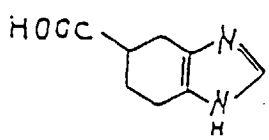

(II)

**2.** Verfahren zur Herstellung eines Tetrahydrobenzimidazolderivats der Formel (Ib):

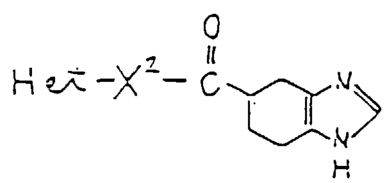

(Ib)

wobei Het wie in Anspruch 1 definiert ist und $X^2$ eine an ein Kohlenstoffatom des heterocyclischen Ringes gebundene Einfachbindung ist, umfassend die Umsetzung einer heterocyclischen Verbindung (IIIa)

$$\text{Het-}X^2\text{-H} \quad \text{(IIIa)}$$

mit Carbonsäure (II) oder einem reaktionsfähigen Derivat davon

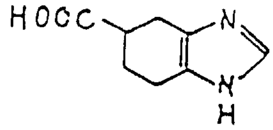

(II)

3. Verfahren zur Herstellung eines Tetrahydrobenzimidazolderivats der Formel (Ic):

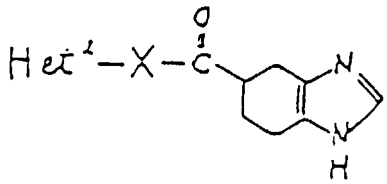

(Ic)

umfassend das N-Alkylieren einer Verbindung (Id)

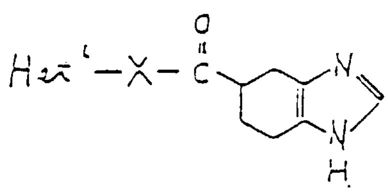

(Id)

wobei $Het^1$ eine Gruppe Het, wie in Anspruch 1 definiert, ist und die -NH- im Ring enthält, X eine Einfachbindung oder -NH-, gebunden an ein Kohlenstoff- oder Stickstoffatom des heterocyclischen Ringes ist, und $Het^2$ $Het^1$ ist, wobei das Ring -NH- umgewandelt ist in Ring

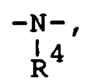

wobei $R^4$ eine $C_1$-$C_6$-Alkyl-, $C_1$-$C_6$-Alkenyl-, $C_1$-$C_6$-ASkinyl-, Cycloalkyl-$C_1$-$C_6$-alkyl- Benzyl- oder Phenethylgruppe ist.

4. Verfahren nach Anspruch 1 oder 3, wobei Het eine stickstoffhaltige heterocyclische Gruppe ist und X eine Einfachbindung ist, die an ein Stickstoffatom des stickstoffhaltigen heterocyclischen Ringes gebunden ist.

5. Verfahren nach einem der vorangehenden Ansprüche, wobei Het

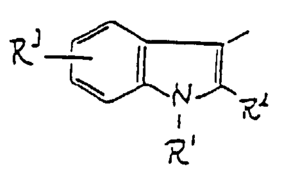

ist, wobei $R^1$ ein Wasserstoffatom oder eine $C_1$-$C_6$-Alkyl-, $C_1$-$C_6$-Alkenyl-, $C_1$-$C_6$-Alkinyl-, cycloalkyl-$C_1$-$C_6$-alkyl-, Benzyl- oder Phenethylgruppe ist, $R^2$ ein Wasserstoffatom oder eine $C_1$-$C_6$-Alkyl-, Benzyl- oder Phenethylgruppe ist, $R^3$ ein Wasserstoff- oder Halogenatom oder eine $C_1$-$C_6$-Alkoxy-, Nitro-, Hydroxyl- oder ($C_1$-$C_6$-Alkoxy)carbo-

nylgruppe ist und X eine Einfachbindung ist.

6. Verfahren nach einem der vorangehenden Ansprüche, umfassend die Umwandlung des Produktes in die Salzform.

7. Verfahren nach Anspruch 1, 2, 3 oder 6, wobei die erhaltene Verbindung ausgewählt ist aus (R)- und (S)-5-[(1-Methylindol-3yl)carbonyl]-4,5,6,7-tetrahydrobenzimidazol,

5-[[1-(2-Propynyl)indol-3-yl]carbonyl]-4,5,6,7-tetrahydrobenzimidazol,
5-[(2,3-Dihydroindol-1-yl)carbonyl]-4,5,6,7-tetrahydrobenzimidazol,
1-[(4,5,6,7-Tetrahydrobenzimidazol-5-yl)carbonyl]-2,3-dihydrobenzimidazol-2-on,
5-[(2-Methylindolizin-3-yl)carbonyl]-4,5,6,7-tetrahydrobenzimidazol,
3-[(Indolizin-3-yl)carbonyl]-4,5,6,7-tetrahydrobenzimidazol,
5-[(1-Methylindolizin-3-yl)carbonyl]4,5,6,7-tetrahydrobenzimidazol und pharmazeutisch annehmbaren Salzen dieser Verbindungen.

8. Verfahren nach Anspruch 1, 2, 3 oder 6, wobei die entstandene Verbindung ausgewählt ist aus 5-[(2-Benzylindol-3-yl)-carbonyl]-4,5,6,7-tetrahydrobenzimidazol, 5-[(1-n-Butylindol-3-yl)carbonyl]-4,5,6,7-tetrahydrobenzimidazol, 5-[(1-cyclohexylmethylindol-3-yl)carbonyl]-4,5,6,7-tetrahydrobenzimidazol, 5-[1-Benzylindol-3-yl)carbonyl]-4,5,6,7-tetrahydrobenzimidazol, N-(Pyridin3-yl)-4,5,6,7-tetrahydrobenzimidazol-5-carboxamid und N-(5,6-Dihydro-4H-cyclopentathiazol-2-yl)-4,5,6,7-tetrahydrobenzimidazol5-carboxamid und pharmazeutisch annehmbaren Salzen davon.

9. Verfahren zur Herstellung eines Arzneimittels, umfassend das Vermischen mindestens einer Verbindung, ausgewählt aus Tetrahydrobenzimidazolderivaten der Formel (I) und pharmazeutisch annehmbaren Salzen davon:

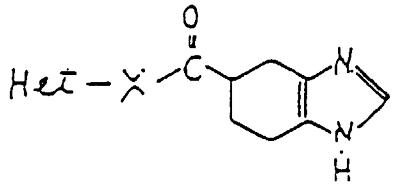

(I)

wobei Het wie in Anspruch 1 definiert ist und X wie in Anspruch 3 definiert ist mit einem pharmazeutisch annehmbaren Träger.

10. Verwendung einer Tetrahydroimidazolverbindung nach Anspruch 9 zur Herstellung eines Arzneimittels mit 5HT3-Antagonistenaktivität.

11. Verwendung einer Tetrahydroimidazolverbindung nach Anspruch 9 zur Herstellung eines Arzneimittels zur Verhütung oder Behandlung von Magen-Darm-Störungen oder zur Unterdrückung von Übelkeit und/oder Erbrechen, das durch Chemotherapie oder Bestrahlung hervorgerufen ist oder zur Verhütung oder Behandlung von Migräne, Cluster headache oder Trigeminusneuralgien oder zur Verhütung oder Behandlung von Angst oder psychischen Störungen.

12. Verwendung einer Tetrahydroimidazolverbindung nach Anspruch 9 zur Herstellung eines Arzneimittels zur Verhütung oder Behandlung von anaphylaktischem Intestinalsyndrom.

**Revendications**

**Revendications pour les Etats contractants suivants : DE, GB, FR, IT, NL, SE, CH, LI, BE, AT, LU, DK, GR**

1. Un dérivé de tétrahydrobenzimidazole de formule (I) ou un sel pharmaceutiquement acceptable de celui-ci :

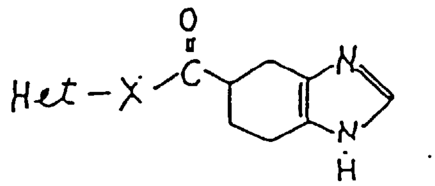

(I)

dans laquelle Het est un groupe hétérocyclique sélectionné parmi la pyrrolidine, la pipéridine, la pipérazine, la morpholine, le pyrrole, le furanne, le thiophène, l'imidazole, l'oxazole, le thiazole, le pyrazole, l'isoxazole, l'isothiazole, le triazole, le thiadiazole, l'oxadiazole, la pyridine, la pyridazine, la pyrimidine, la pyrazine, le 4H-cyclopentathiazole, l'indole, l'isoindole, le 2,3-dihydroindole (indoline), l'isoindoline, l'hydroxyindole, l'indazole, l'indolizine, le benzothiophène, le benzofuranne, le benzothiazole, le benzimidazole, le benzoxazole, le 4,5,6,7tétrahydrobenzothiophène, le 2,3-dihydrobenzimidazol-2-one, la quinoléine, l'isoquinoléine, la 1,2,3,4-tétrahydroquinoléine, la 1,2,3,4-tétrahydroisoquinoléine, la 1,4-benzoxazine, la phénothiazine, le carbazole et la β-carboline, lequel groupe hétérocyclique peut être substitué avec 1 à 3 substituants sélectionnés parmi les groupes alkyle en $C_1$-$C_6$ , alkényle en $C_1$-$C_6$, alkynyle en $C_1$-$C_6$, cycloalkyl-$C_1$-$C_6$ alkyle, benzyle, phénéthyle, alcoxy en $C_1$-$C_6$, nitro, hydroxyle et ($C_1$-$C_6$ alcoxy)carbonyle et des atomes d'halogène;
et X est une liaison simple ou -NH- qui est liée à un atome de carbone ou d'azote du cycle hétérocyclique.

2. Un composé tel que revendiqué à la revendication 1, dans lequel Het est

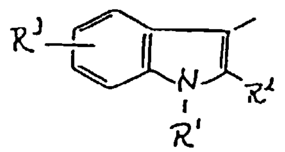

où $R^1$ est un atome d'hydrogène ou un groupe alkyle en $C_1$-$C_6$, alkényle en $C_1$-$C_6$, alkynyle en $C_1$-$C_6$, cycloalkyl-$C_1$-$C_6$ alkyle, benzyle, ou phénéthyle ; $R^2$ est un atome d'hydrogène ou un groupe alkyle en $C_1$-$C_6$, benzyle ou phénéthyle ; $R^3$ est un atome d'hydrogène ou d'halogène ou un groupe alcoxy en $C_1$-$C_6$, nitro, hydroxyle ou ($C_1$-$C_6$ alcoxy)carbonyle ;
et X est une liaison simple.

3. Un composé tel que revendiqué à la revendication 1 ou 2, dans lequel Het est un groupe hétérocyclique contenant de l'azote ; et X est une liaison simple reliée à un atome d'azote du cycle hétérocyclique contenant de l'azote.

4. Un composé ou plus selon la revendication 1, sélectionnés parmi le (R)- et (S)-5-[(1-méthylindol-3-yl)carbonyl]-4,5,6,7-tétrahydrobenzimidazole,

le 5-[(1-(2-propynyl)indol-3-yl)carbonyl]-4,5,6,7tétrahydrobenzimidazole,

le 5-[(2,3-dihydroindol-1-yl)carbonyl]-4,5,6,7-tétrahydrobenzimidazole,

le 1-[(4,5,6,7-tétrahydrobenzimidazol-5-yl]carbonyl)-2,3-dihydrobenzimidazol-2-one;

le 5-[(2-méthylindolizin-3-yl)carbonyl]-4,5,6,7- j tétrahydrobenzimidazole,

le 3-[(indozilin-3-yl)carbonyl]-4,5,6,7tétrahydrobenzimidazole,

le 5-[(1-méthylindolizin-3-yl)carbonyl]-4,5,6,7tétrahydrobenzimidazole; et des sels pharmaceutiquement acceptables desdits composés.

5. Un composé ou plus selon la revendication 1, sélectionnés parmi le 5-[(2-benzylindol-3-yl)-carbonyl]-4,5,6,7tétrahydrobenzimidazole ; le 5-[(1-n-butylindol-3-yl)carbonyl]4,5,6,7-tétrahydrobenzimidazole; le 5-[(1-cyclohexylméthylindol-3-yl)carbonyl]-4,5,6,7-tétrahydrobenzimidazole ; le 5-[(1-benzylindol-3-yl) carbonyl] 4,5,6,7-tétrahydrobenzimidazole; le N-(pyridin-3-yl)-4,5,6,7-tétrahydrobenzimidazole-5-carboxamide; et le N-(5,6-dihydro-4H-cyclopentathiazol-2-yl)-4,5,6,7-tétrahydrobenzimidazole-5carboxamide ; et des sels pharmaceutiquement acceptables de ceux-ci.

**6.** Un procédé pour préparer un dérivé de tétrahydrobenzimidazole de formule (Ia) :

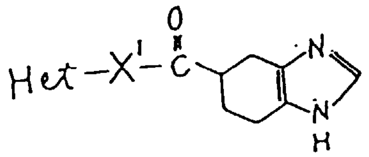

(Ia)

dans laquelle Het est tel que défini à la revendication 1 et $X^1$ est une liaison simple reliée à un atome d'azote du cycle hétérocyclique ou reliée en -NH- à un atome de carbone du cycle hétérocyclique,
qui comprend la réaction d'une amine, d'un amide ou d'une urée (III)

$$Het\text{-}X^1\text{-}H \qquad (III)$$

avec un acide carboxylique (II) ou un dérivé réactif de celui-ci

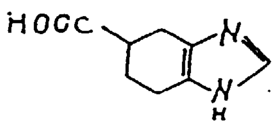

(II)

**7.** Un procédé pour préparer un dérivé de tétrahydrobenzimidazole de formule (Ib) :

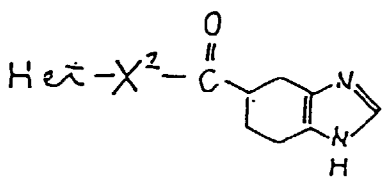

(Ib)

dans laquelle Het est tel que défini à la revendication 1 et $X^2$ est une liaison simple reliée à un atome de carbone du cycle hétérocyclique,
qui comprend la réaction d'un composé hétérocyclique (IIIa)

$$Het\text{-}X^2\text{-}H \qquad (IIIa)$$

avec un acide carboxylique (II) ou un dérivé réactif de celui-ci.

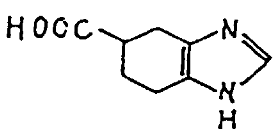

(II)

**8.** Un procédé pour préparer un dérivé de tétrahydrobenzimidazole de formule (Ic) :

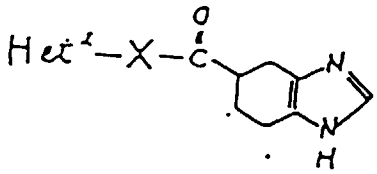

(Ic)

qui comprend la N-alkylation d'un composé (Id)

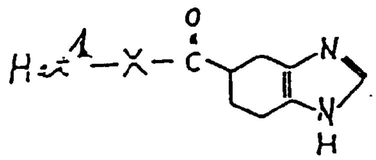

(Id)

où $Het^1$ est un groupe Het qui est tel que défini à la revendication 1 et qui a -NH- dans son cycle, X est tel que défini à la revendication 1, et $Het^2$ est $Het^1$ dans lequel ledit cycle -NH- a été converti en un cycle

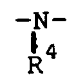

où $R^4$ est un groupe alkyle en $C_1$-$C_6$, alkényle en $C_1$-$C_6$, alkynyle en $C_1$-$C_6$, cycloalkyl- $C_1$-$C_6$ alkyle, benzyle ou phénéthyle.

**9.** Un procédé selon l'une quelconque des revendications 6 à 8 incluant la conversion du produit sous la forme d'un sel.

**10.** Une composition pharmaceutique contenant au moins un composé selon l'une quelconque des revendications 1 à 5 avec un support pharmaceutiquement acceptable.

**11.** Une composition selon la revendication 10 pour l'utilisation en tant qu'antagoniste du $5HT_3$.

**12.** Une composition selon la revendication 10 pour la prévention ou le traitement d'un trouble gastrointestinal; ou pour supprimer les nausées et/ou les vomissements induits par une chimiothérapie ou des radiations ; ou pour prévenir ou traiter des migraines, des maux de tête ou des névralgies faciales ; ou pour prévenir ou traiter des anxiétés ou des troubles psychotiques.

**13.** Une composition selon la revendication 10 pour la prévention ou le traitement des syndromes intestinaux anaphylactiques.

**14.** L'utilisation d'un composé selon l'une quelconque des revendications 1 à 5 pour la préparation d'un médicament ayant une activité antagoniste du $5HT_3$.

**15.** L'utilisation d'un composé selon l'une quelconque des revendications 1 à 5 pour la préparation d'un médicament pour la prévention ou le traitement de troubles gastrointestinaux ; ou pour supprimer des nausées et des vomissements induits par une chimiothérapie ou des radiations ; ou pour prévenir ou traiter des migraines, des maux de tête ou des névralgies faciales ; ou pour prévenir ou traiter des anxiétés ou des troubles psychotiques.

**16.** L'utilisation d'un composé selon l'une quelconque des revendications 1 à 5 pour la préparation d'un médicament pour la prévention ou le traitement de syndromes intestinaux anaphylactiques.

**Revendications pour l'Etat contractant suivant : ES**

**1.** Un procédé pour préparer un dérivé de tétrahydrobenzimidazole de formule (Ia) :

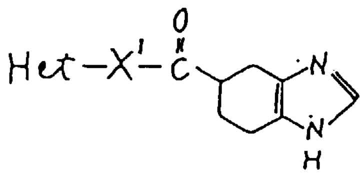

(Ia)

dans laquelle Het est un groupe hétérocyclique sélectionné parmi la pyrrolidine, la pipéridine, la pipérazine, la morpholine, le pyrrole, le furanne, le thiophène, l'imidazole, l'oxazole, le thiazole, le pyrazole, l'isoxazole, l'isothiazole, le triazole, le thiadiazole, l'oxadiazole, la pyridine, la pyridazine, la pyrimidine, la pyrazine, le 4H-cyclopentathiazole, l'indole, l'isoindole, le 2,3-dihydroindole (indoline), l'isoindoline, l'hydroxyindole, l'indazole, l'indolizine, le benzothiophène, le benzofuranne, le benzothiazole, le benzimidazole, le benzoxazole, le 4,5,6,7tétrahydrobenzothiophène, le 2,3-dihydrobenzimidazol-2-one, la quinoléine, l'isoquinoléine, la 1,2,3,4-tétrahydroquinoléine, la 1,2,3,4-tétrahydroisoquinoléine, la 1,4-benzoxazine, la phénothiazine, le carbazole et la β-carboline, lequel groupe hétérocyclique peut être substitué avec 1 à 3 substituants sélectionnés parmi les groupes alkyle en $C_1$-$C_6$, alkényle en $C_1$-$C_6$, alkynyle en $C_1$-$C_6$, cycloalkyl-$C_1$-$C_6$ alkyle, le benzyle, le phénéthyle, alcoxy en $C_1$-$C_6$, nitro, hydroxyle, et ($C_1$-$C_6$ alcoxy)carbonyle et des atomes d'halogène;
et $X^1$ est une liaison simple reliée à un atome d'azote du cycle hétérocyclique ou reliée en -NH- à un atome de carbone du cycle hétérocyclique,
qui comprend la réaction d'une amine, d'un amide ou d'une urée (III)

$$\text{Het-}X^1\text{-H} \qquad \text{(III)}$$

avec un acide carboxylique (II) ou un dérivé réactif de celui-ci

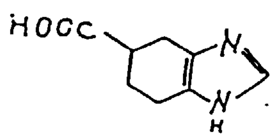

(II)

**2.** Un procédé pour préparer un dérivé de tétrahydrobenzimidazole de formule (Ib) :

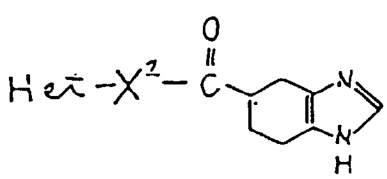

(Ib)

dans laquelle Het est tel que défini à la revendication 1 et $X^2$ est une liaison simple reliée à un atome de carbone du cycle hétérocyclique,
qui comprend la réaction d'un composé hétérocyclique (IIIa)

$$\text{Het-}X^2\text{-H} \qquad \text{(IIIa)}$$

avec un acide carboxylique (II) ou un dérivé réactif de celui-ci.

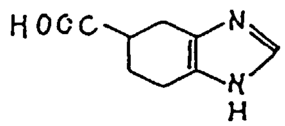

(II)

3. Un procédé pour préparer un dérivé de tétrahydrobenzimidazole de formule (Ic) :

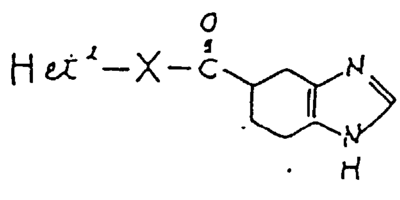

(Ic)

qui comprend la N-alkylation d'un composé (Id)

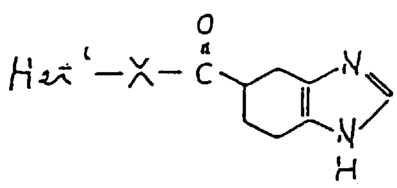

(Id)

où $Het^1$ est un groupe Het qui est tel que défini à la revendication 1 et qui a -NH- dans son cycle, X est une liaison simple ou -NH- qui est liée à un atome de carbone ou d'azote du cycle hétérocyclique, et $Het^2$ est $Het^1$ dans lequel ledit cycle -NH- a été converti en un cycle

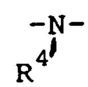

où $R^4$ est un groupe alkyle en $C_1$-$C_6$, alkényle en $C_1$-$C_6$, alkynyle en $C_1$-$C_6$, cycloalkyl- en $C_1$-$C_6$ alkyl, benzyle ou phénéthyle.

4. Un procédé tel que revendiqué dans la revendication 1 ou 3, dans lequel Het est un groupe hétérocyclique contenant de l'azote et X est une liaison simple reliée à un atome d'azote du cycle hétérocyclique contenant de l'azote.

5. Un procédé tel que revendiqué à l'une quelconque des revendications précédentes, dans lequel Het est

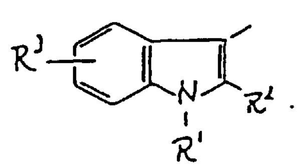

où $R^1$ est un atome d'hydrogène ou un groupe alkyle en $C_1$-$C_6$, alkényle en $C_1$-$C_6$, alkynyle en $C_1$-$C_6$, cycloalkyl-$C_1$-$C_6$ alkyle, benzyle, ou phénéthyle ; $R^2$ est un atome d'hydrogène ou un groupe alkyle en $C_1$-$C_6$, benzyle ou phénéthyle ; $R^3$ est un atome d'hydrogène ou d'halogène ou un groupe alcoxy en $C_1$-$C_6$, nitro, hydroxyle ou ($C_1$-$C_6$ alcoxy)carbonyle ;
et X est une liaison simple.

**6.** Un procédé selon l'une quelconque des revendications précédentes, incluant la conversion du produit sous la forme d'un sel.

**7.** Un procédé selon la revendication 1, 2, 3 ou 6, dans lequel le composé de produit est sélectionné parmi le (R)- et (S)-5-[(l-méthylindol-3-yl)carbonyl]-4,5,6,7-tétrahydrobenzimidazole,

le 5-[(1-(2-propynyl)indol-3-yl)carbonyl]-4,5,6,7-tétrahydrobenzimidazole,

le 5-[(2,3-dihydroindol-1-yl)carbonyl]-4,5,6,7tétrahydrobenzimidazole,

le 1-[(4,5,6,7-tétrahydrobenzimidazol-5-yl)carbonyl]-2,3-dihydrobenzimidazol-2-one;

le 5-[(2-méthylindolizin-3-yl)carbonyl]-4,5,6,7-tétrahydrobenzimidazole,

le 3-[(indozilin-3-yl)carbonyl] -4,5,6,7-tétrahydrobenzimidazole,

le 5-[(1-méthylindolizin-3-yl)carbonyl)-4,5,6,7-tétrahydrobenzimidazole; et des sels pharmaceutiquement acceptables desdits composés.

**8.** Un procédé selon la revendication 1, 2, 3 ou 6, dans lequel le composé de produit est sélectionné parmi le 5-[(2-benzylindol-3-yl)-carbonyl]-4,5,6,7-tétrahydrobenzimidazole ; le 5-[(1-n-butylindol-3-yl)carbonyl]-4,5,6,7-tétrahydrobenzimidazole ; le 5-[(1-cyclohexylméthylindol-3-yl)carbonyl]-4,5,6,7-tétrahydrobenzimidazole; le 5-[(1-benzylindol-3-yl)carbonyl]-4,5,6,7-tétrahydrobenzimidazole; le N-(pyridin-3-yl)-4,5,6,7-tétrahydrobenzimidazole-5-carboxamide ; et le N-(5,6-dihydro-4H-cyclopentathiazol-2-yl)-4,5,6,7-tétrahydrobenzimidazole-5-carboxamide ; et des sels pharmaceutiquement acceptables de ceux-ci.

**9.** Un procédé de fabrication d'une composition pharmaceutique qui comprend le mélange avec un support pharmaceutiquement acceptable d'au moins un composé sélectionné parmi un dérivé de tétrahydrobenzimidazole de formule (I) et un sel pharmaceutiquement acceptable de celui-ci :

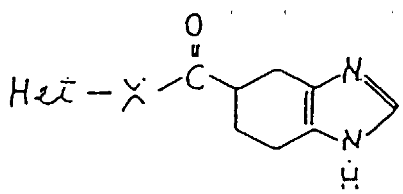

(I)

dans laquelle Het est tel que défini à la revendication 1 et X est tel que défini à larevendication 3.

**10.** L'utilisation d'un composé de tétrahydroimidazole tel que défini à la revendication 9 pour la préparation d'un médicament ayant une activité antagoniste du $5HT_3$.

**11.** L'utilisation d'un composé de tétrahydroimidazole tel que défini à la revendication 9 pour la préparation d'un médicament pour la prévention ou le traitement des troubles gastrointestinaux ou pour supprimer des nausées et des vomissements induits par une chimiothérapie ou des radiations ; ou pour prévenir ou traiter des migraines, des maux de tête ou des névralgies faciales ; ou pour prévenir ou traiter des anxiétés ou des troubles psychotiques.

**12.** L'utilisation d'un composé de tétrahydroimidazole tel que défini à la revendication 9 pour la préparation d'un médicament pour la prévention ou le traitement de syndromes intestinaux anaphylactiques.